# EUROPEAN PATENT APPLICATION

(11) **EP 4 786 582 A1**
(43) Date of publication of application: **05.08.2026**
(21) Application number: 24872601.0
(22) Date of filing: 30.09.2024
(51) Int. Cl.: C12N 5/10, C12N 15/09, C12N 15/12

(54) **HLA GENE MODIFIED CELL**

(30) Priority: 29.09.2023 JP 2023170195
(71) Applicant: AJINOMOTO CO., INC., Chuo-ku Tokyo 104-8315 (JP); Logomix, Inc., Tokyo 104-0053 (JP)
(72) Inventor: MATSUMOTO, Takuya, Kawasaki-shi, Kanagawa 210-8681 (JP); KONISHI, Atsushi, Kawasaki-shi, Kanagawa 210-8681 (JP); OKAMOTO, Aika, Kawasaki-shi, Kanagawa 210-8681 (JP); YOSHIDA, Myu, Yokohama-shi, Kanagawa 226-0026 (JP); AIZAWA, Yasunori, Yokohama-shi, Kanagawa 226-0026 (JP); MATSUZAWA, Ayumi, Yokohama-shi, Kanagawa 226-0026 (JP)
(74) Representative: Hoffmann Eitle
(86) International application number: PCT/JP2024/034995
(87) International publication number: WO 2025/070822

(57) **Abstract**

The present invention provides a cell having improved histocompatibility with a transplant recipient. Specifically, by knocking in a polynucleotide encoding a single-chain fusion peptide mimicking HLA-G and/or HLA-E that functions in an inhibitory manner against the activity (cytotoxicity) of NK cells or macrophages into a region encoding an alpha chain of an HLA Class I molecule, the expression of the alpha chain of the HLA Class I molecule is suppressed while the single-chain fusion peptide is expressed.

## Description

### Technical Field

The present invention relates to an HLA gene-modified cell having improved histocompatibility with a transplant recipient.

### Background Art

In cell-based regenerative medicine, functions of organs or tissues damaged by diseases, injuries, or the like are repaired and regenerated by transplanting cells into a patient's body.

Immune rejection reactions that occur when HLA (Human Leukocyte Antigen) does not match between transplanted cells and a patient significantly affect the success or failure of the treatment.

Autologous transplantation using cells collected from the patient themselves is excellent in terms of suppression of immune rejection reactions; however, the production cost of cells for transplantation becomes high.

Allogeneic transplantation using cells of another person is excellent in that the production cost of cells for transplantation is low; however, measures for suppressing immune rejection reactions (administration of immunosuppressants, etc.) are required.

In allogeneic transplantation using iPS cells, a strategy is conceivable in which a plurality of types of cells for transplantation are manufactured in advance from a plurality of types of iPS cell lines obtained from an iPS cell bank, and cells having high HLA compatibility with a patient are selected. However, this strategy also has problems that the manufacturing cost increases depending on the number of lines of iPS cells to be used, and that it is difficult to cope with HLA types of all patients with the cells for transplantation manufactured in advance.

The immune rejection reaction occurs when immune cells (T cells, etc.) of a patient recognize transplanted cells having an HLA type different from that of the patient as non-self and attack them.

Therefore, a technique (Hypoimmunogenic technique) for improving histocompatibility with a transplant recipient by modifying genes of transplanted cells to suppress the expression of HLA is attracting attention.

By applying the Hypoimmunogenic technique to one type of iPS cell, it is possible to manufacture cells for transplantation applicable to diverse patients at low cost.

Patent Literature 1 discloses a stem cell in which expression of HLA Class I molecules (HLA-A, HLA-B and HLA-C) and HLA Class II molecules is reduced by gene modification.

Patent Literature 2 discloses a primate cell in which beta-2 microglobulin (B2M) is deficient due to gene modification. In a cell deficient in B2M which is a constituent component (beta chain) of an HLA Class I molecule, an HLA Class I molecule capable of antigen presentation is not formed.

Furthermore, Patent Literature 2 discloses that, in creating a B2M-deficient cell, a polynucleotide encoding (1) a single-chain fusion protein (heterodimer protein) comprising B2M and an alpha chain of an HLA Class I molecule or (2) a single-chain fusion protein (heterotrimer protein) comprising a peptide presented on the cell surface by an HLA Class I molecule (for example, a signal peptide of HLA-G), B2M, and an alpha chain of an HLA Class I molecule is knocked into a B2M locus.

Non Patent Literature 1 discloses that when a polynucleotide encoding (1) a single-chain fusion protein comprising B2M and an alpha chain of HLA-E or (2) a single-chain fusion protein comprising a peptide antigen, B2M, and an alpha chain of HLA-E was knocked into a B2M locus of a human pluripotent stem cell to forcibly express HLA-E, resistance to natural killer (NK) cell-mediated lysis was shown.

However, since B2M is related to expression and functions of various cell surface molecules other than the expression of HLA Class I molecules, there was a problem that knocking out B2M could affect the functions of the cell.

When HLA-G binds to KIR2DL4 and LILRB1 expressed as inhibitory receptors on NK cells or macrophages, their cytotoxicity is suppressed. When HLA-E binds to CD94/NKG2A or CD94/NKG2B expressed as inhibitory receptors on NK cells or macrophages, their cytotoxicity is suppressed (Non Patent Literatures 2 and 3).

### Citation List

### Patent Literatures

Patent Literature 1: JP 2022-526218 A
Patent Literature 2: JP 2014-513948 A

### Non Patent Literatures

Non Patent Literature 1: Nature Biotechnology volume 35, pages 765-772 (2017)
Non Patent Literature 2: Arch Immunol Ther Exp (Warsz). 2016; 64(6): 505-514.
Non Patent Literature 3: Nature. 1998; 391: 795-799

### Summary of Invention

### Problems to be solved by the invention

It was set as an object to provide a cell with improved histocompatibility with a transplant recipient.

### Means for solution of the problems

By knocking in a polynucleotide encoding an effector cell inhibitory factor (particularly a single-chain fusion peptide mimicking HLA-G or HLA-E) that functions in an inhibitory manner against the activity (cytotoxicity) of effector cells (particularly NK cells or macrophages) into a region encoding an alpha chain of an HLA Class I molecule, the expression of the alpha chain of the HLA Class I molecule is suppressed while the effector cell inhibitory factor (particularly the single-chain fusion peptide) is expressed. That is, the present invention relates to the following [1] to [47].
[1] An HLA gene-modified cell, wherein the following polynucleotides (1) and (2) are knocked into a region containing alpha chain genes of HLA-A, HLA-B, HLA-C, HLA-F and HLA-G:
   (1) a polynucleotide encoding a single-chain dimer (SCD) fusion peptide comprising beta-2 microglobulin and an alpha chain of HLA-G;
   (2) a polynucleotide encoding a single-chain trimer (SCT) fusion peptide comprising a signal peptide of HLA-A, HLA-B, HLA-C or HLA-G, beta-2 microglobulin, and an alpha chain of HLA-E.
[2] The cell according to [1], wherein expression of the alpha chain genes of HLA-A, HLA-B, HLA-C, HLA-F and HLA-G is reduced compared with a wild-type cell.
[3] The cell according to [1], wherein the alpha chain genes of HLA-A, HLA-B, HLA-C, HLA-F and HLA-G of the cell before gene modification are deleted.
[4] The cell according to [1], wherein
   (1) the polynucleotide encoding the SCD is knocked into a region containing alpha chain genes of HLA-F, HLA-G and HLA-A, and
   (2) the polynucleotide encoding the SCT is knocked into a region containing alpha chain genes of HLA-C and HLA-B.
[5] The cell according to [1], wherein
   (2) the polynucleotide encoding the SCT is knocked into a region containing alpha chain genes of HLA-F, HLA-G and HLA-A, and
   (1) the polynucleotide encoding the SCD is knocked into a region containing alpha chain genes of HLA-C and HLA-B.
[6] An HLA gene-modified cell, wherein the following polynucleotide (1) is knocked into a region containing alpha chain genes of HLA-F, HLA-G and HLA-A:
   (1) a polynucleotide encoding a single-chain dimer (SCD) fusion peptide comprising beta-2 microglobulin and an alpha chain of HLA-G.
[7] The cell according to [6], wherein expression of the alpha chain genes of HLA-F, HLA-G and HLA-A is reduced compared with a wild-type cell.
[8] The cell according to [6], wherein the alpha chain genes of HLA-F, HLA-G and HLA-A of the cell before gene modification are deleted.
[9] An HLA gene-modified cell, wherein the following polynucleotide (2) is knocked into a region containing alpha chain genes of HLA-C and HLA-B:
   (2) a polynucleotide encoding a single-chain trimer (SCT) fusion peptide comprising a signal peptide of HLA-A, HLA-B, HLA-C or HLA-G, beta-2 microglobulin, and an alpha chain of HLA-E.
[10] The cell according to [9], wherein expression of the alpha chain genes of HLA-C and HLA-B is reduced compared with a wild-type cell.
[11] The cell according to [9], wherein the alpha chain genes of HLA-C and HLA-B of the cell before gene modification are deleted.
[12] The cell according to [1] or [6], wherein the region containing alpha chain genes of HLA-F, HLA-G and HLA-A is a chromosomal region corresponding to a chromosomal region of chr6: 29,704,906-29,958,642 of hg38 genome sequence.
[13] The cell according to [1] or [9], wherein the region containing alpha chain genes of HLA-C and HLA-B is a chromosomal region corresponding to a chromosomal region of chr6: 31,224,837-31,378,196 of hg38 genome sequence.
[14] The cell according to [1] or [9], wherein the region containing alpha chain genes of HLA-C and HLA-B is a chromosomal region corresponding to a chromosomal region of chr6: 31,227,286-31,378,780 of hg38 genome sequence.
[15] The cell according to [1], [6] or [9], wherein (1) the polynucleotide encoding the SCD and/or (2) the polynucleotide encoding the SCT is knocked into only one allele of the subject cell.
[16] The cell according to [1], [6] or [9], wherein expression of an HLA Class II molecule gene is further reduced compared with a wild-type cell by modification.
[17] The cell according to [1], [6] or [9], wherein an HLA Class II molecule gene is further deleted by modification.
[18] The cell according to [16], wherein the HLA Class II molecule gene is a CIITA gene.
[19] The cell according to [17], wherein the HLA Class II molecule gene is a CIITA gene.
[20] The cell according to [1], [6] or [9], which is a human pluripotent stem cell.
[21] The cell according to [1], [6] or [9], which is a human iPS cell or ES cell.
[22] The cell according to [1], [6] or [9], which is a human iPS cell line 253G4 or a human iPS cell line 201B7.
[23] The cell according to [1], [6] or [9], which is a cell obtained by differentiating a human pluripotent stem cell.
[24] The cell according to [23], wherein the human pluripotent stem cell is a human iPS cell or ES cell.
[25] The cell according to [23], wherein the human pluripotent stem cell is a human iPS cell line 253G4, a human iPS cell, or an ES cell.
[26] The cell according to [23], wherein the cell obtained by differentiating a human pluripotent stem cell is an immune cell, a tissue stem cell, or a somatic cell.
[27] The cell according to [26], wherein the immune cell is a T cell or an NK cell.
[28] The cell according to [1] or [6], wherein the SCD comprises an amino acid sequence set forth in SEQ ID NO: 1 or 4, or an amino acid sequence having 80% or more sequence identity with the amino acid sequence set forth in SEQ ID NO: 1 or 4.
[29] The cell according to [1] or [9], wherein the SCT comprises an amino acid sequence set forth in SEQ ID NO: 6, or an amino acid sequence having 80% or more sequence identity with the amino acid sequence set forth in SEQ ID NO: 6.
[30] The cell according to [1] or [6], wherein the polynucleotide of (1) comprises a nucleotide sequence set forth in SEQ ID NO: 2 or 5, or a nucleotide sequence having 80% or more sequence identity with the nucleotide sequence set forth in SEQ ID NO: 2 or 5.
[31] The cell according to [1] or [9], wherein the polynucleotide of (2) comprises a nucleotide sequence set forth in SEQ ID NO: 7, or a nucleotide sequence having 80% or more sequence identity with the nucleotide sequence set forth in SEQ ID NO: 7.
[32] The cell according to [1] or [6], wherein the SCD further comprises a linker sequence between the beta-2 microglobulin and the alpha chain of HLA-G.
[33] The cell according to [1] or [9], wherein the SCT further comprises a linker sequence between the signal peptide and the beta-2 microglobulin.
[34] The cell according to [1] or [9], wherein the SCT further comprises a linker sequence between the beta-2 microglobulin and the alpha chain of HLA-E.
[35] The cell according to [32], wherein the linker sequence comprises a peptide consisting of glycine and serine.
[36] The cell according to [33], wherein the linker sequence comprises a peptide consisting of glycine and serine.
[37] The cell according to [34], wherein the linker sequence comprises a peptide consisting of glycine and serine.
[38] The cell according to [32], wherein the linker sequence comprises an amino acid sequence set forth in SEQ ID NO: 3.
[39] The cell according to [33], wherein the linker sequence comprises an amino acid sequence set forth in SEQ ID NO: 8.
[40] The cell according to [34], wherein the linker sequence comprises an amino acid sequence set forth in SEQ ID NO: 9.
[41] The cell according to [1] or [6], wherein a promoter sequence of an alpha chain gene of HLA-A is knocked in together with the polynucleotide of (1), and the promoter sequence is operably linked to a sequence encoding the SCD.
[42] The cell according to [41], wherein the promoter sequence of the alpha chain gene of HLA-A comprises a nucleotide sequence set forth in SEQ ID NO: 10, or a nucleotide sequence having 80% or more sequence identity with the nucleotide sequence set forth in SEQ ID NO: 10.
[43] The cell according to [1] or [9], wherein a promoter sequence of an alpha chain gene of HLA-B is knocked in together with the polynucleotide of (2), and the promoter sequence is operably linked to a sequence encoding the SCT.
[44] The cell according to [43], wherein the promoter sequence of the alpha chain gene of HLA-B comprises a nucleotide sequence set forth in SEQ ID NO: 11, or a nucleotide sequence having 80% or more sequence identity with the nucleotide sequence set forth in SEQ ID NO: 11.
[45] The cell according to [1], [6] or [9], wherein rejection reaction by T cells and/or NK cells is alleviated compared with a wild-type cell.
[46] The cell according to [45], wherein the T cells are CD8⁺ T cells and/or CD4⁺ T cells.
[47] A cell derived from the HLA gene-modified cell according to [1], [6] or [9]. Advantageous Effects of Invention

In the cell of the present invention, expression of an alpha chain of a predetermined HLA Class I molecule (and thus expression of a functional HLA Class I molecule having antigen-presenting ability) is suppressed; therefore, attacks from T cells or the like of a transplant recipient are avoided. Furthermore, in the cell of the present invention, attacks from NK cells or macrophages are avoided by expression of the effector cell inhibitory factor (particularly the single-chain fusion peptide). The cell of the present invention, in which histocompatibility with a transplant recipient is improved by these mechanisms, is useful as a cell for transplantation for regenerative medicine (particularly allogeneic transplantation).

In modification of an HLA gene using a Cre/loxP system (Patent Literature 2), a foreign gene used as a tool (loxP sequence, marker gene, etc.) may remain in the genome after modification, which is unfavorable for transplantation purposes. In the present invention, the cell of the present invention can be manufactured with high efficiency using a homologous recombination method using a site-specific nuclease (described later) without using a loxP sequence and further without leaving traces of a marker gene.

### Brief Description of Drawings

Fig. 1 shows expression analysis results of an SCD coding sequence in the gene-modified cell of Example 1.
Fig. 2 shows expression analysis results of HLA-A, HLA-B, HLA-C, SCT and SCD in the gene-modified cell of Example 2.
Fig. 3 shows T cell responsiveness to the gene-modified cell of Example 2.
Fig. 4 shows T cell cytotoxicity against the gene-modified cell of Example 2.
Fig. 5 shows NK cell cytotoxicity against the gene-modified cell of Example 2.

### Description of Embodiments

### [Cells to be subjected to gene modification]

The gene modification according to the present invention can be applied to cells having HLA genes without particular limitation. The cells may have a haploid or larger genome, and diploid (cells having two alleles) are preferred.

The cells may be mammalian (e.g., human) cells, or non-human mammalian (e.g., monkey, dog, cat, bovine, horse, sheep, goat, llama, rodent) or other vertebrate cells, and can be appropriately selected according to the transplant recipient. The cells may be either proliferative or non-proliferative cells, but proliferative cells are preferred.

The non-proliferative cells include nerve cells and cardiomyocytes.

The proliferative cells include pluripotent stem cells (iPS cells or embryonic stem cells (ES cells)), hematopoietic stem cells, T cells, NK cells, and B cells, and iPS cells are preferred. In a preferred embodiment, the cells to be subjected to gene modification (subject cells) are human iPS cell lines available from HLA homozygous donor-derived iPS cell stocks of Kyoto University iPS Cell Research Foundation (for example, human iPS cell line 253G1, human iPS cell line 253G4, human iPS cell line 201B7, human iPS cell line 409B2, human iPS cell line 454E2, human iPS cell line 585A1, human iPS cell line 585B1, human iPS cell line 606A1, human iPS cell line 610B1, human iPS cell line 648A1, human iPS cell line 1201C1, human iPS cell line 1210B2, human iPS cell line 1231A3, human iPS cell line 1383D2 or human iPS cell line 1383D6, HiPS-RIKEN-1A line, HiPS-RIKEN-2A line, HiPS-RIKEN-12A line or Nips-B2 line). In a further preferred embodiment, the cells to be subjected to gene modification are the human iPS cell line 253G4 or the human iPS cell line 201B7.

The cells may be differentiated cells in which expression of HLA is considered to be higher than in undifferentiated cells (iPS cells, etc.).

The cells may be cells obtained by differentiating pluripotent stem cells (for example, human T cells or NK cells obtained by differentiating human pluripotent stem cells).

The differentiated cells or the cells obtained by differentiating pluripotent stem cells may be, for example, immune cells such as T cells, NK cells, macrophages, B cells, dendritic cells, monocytes, eosinophils, neutrophils, basophils, innate lymphoid cells, and mast cells; tissue stem cells such as hematopoietic stem cells, mesenchymal stem cells (including bone marrow-derived or adipose-derived mesenchymal stem cells), and neural stem cells; or somatic cells such as adipocytes, epidermal cells, endothelial cells, chondrocytes, skeletal myoblasts, retinal cells, retinal pigment epithelial cells, corneal epithelial cells, fibroblasts, muscle cells, cardiomyocytes, nerve cells, astrocytes, hepatocytes, pancreatic beta cells, kidney cells, and adrenal cells; or precursor cells thereof.

### [Single-chain fusion peptide mimicking HLA-G or HLA-E]

The single-chain fusion peptide is configured to exhibit a function possessed by HLA-G or HLA-E (a function of binding to an inhibitory receptor expressed on the surface of NK cells or macrophages).

HLA-G is a ligand for inhibitory receptors KIR2DL4 and LILRB1. HLA-E is a ligand for inhibitory receptors CD94/NKG2A and CD94/NKG2B.

In the present invention, in order to obtain a suppression effect on cytotoxicity of NK cells or macrophages mediated by the inhibitory receptor, the single-chain fusion peptide is introduced into cells and expressed.

The single-chain fusion peptide mimicking HLA-G binds to the inhibitory receptor KIR2DL4 or LILRB1, thereby suppressing cytotoxicity of NK cells or macrophages mediated by the inhibitory receptor.

The single-chain fusion peptide mimicking HLA-E binds to the inhibitory receptor CD94/NKG2A or CD94/NKG2B, thereby suppressing cytotoxicity of NK cells or macrophages mediated by the inhibitory receptor.

Since HLA-G and HLA-E are both HLA Class I molecules, three constituent components (alpha chain, beta-2 microglobulin, and endogenous peptide presented on each HLA molecule) are required for their functions to be exhibited.

The single-chain fusion peptide is preferably at least one kind selected from a single-chain fusion peptide comprising beta-2 microglobulin (hereinafter, also referred to as B2M) and an alpha chain of HLA-G, and a single-chain fusion peptide comprising beta-2 microglobulin and an alpha chain of HLA-E.

The single-chain fusion peptide may be a single-chain fusion peptide further comprising an endogenous peptide presented on an HLA molecule.

### [Single-chain fusion peptide mimicking HLA-G (SCD)]

The single-chain fusion peptide mimicking HLA-G is a single-chain dimer (SCD) fusion peptide comprising "beta-2 microglobulin (hereinafter, also referred to as B2M)" and "alpha chain of HLA-G." Note that, unlike the single-chain fusion peptide mimicking HLA-G (SCT) described later, an endogenous peptide presented on the SCD is supplied from inside the cell.

In the SCD, it is preferable that "B2M" and "alpha chain of HLA-G" are present in this order from the N-terminal side toward the C-terminal side.

In the present invention, a known amino acid sequence of B2M can be used. The amino acid sequence of B2M is preferably a wild-type sequence, but one or a plurality of amino acids may be deleted, substituted or added as long as the function of the SCD is not impaired.

In the present invention, a known amino acid sequence of the alpha chain of HLA-G can be used. The amino acid sequence of the alpha chain of HLA-G is preferably a wild-type sequence, but one or a plurality of amino acids may be deleted, substituted or added as long as the function of the SCD is not impaired.

In a preferred embodiment, the amino acid sequence of the SCD may be a sequence comprising a sequence having 80% or more, 85% or more, 90% or more, 95% or more, or 97% or more sequence identity with the following SEQ ID NO: 1.

In a particularly preferred embodiment, the amino acid sequence of the SCD is the sequence set forth in SEQ ID NO: 1 below. In SEQ ID NO: 1, the 1st to 119th amino acid sequence corresponds to B2M, the 120th to 134th amino acid sequence corresponds to a linker, and the 135th to 448th amino acid sequence corresponds to the alpha chain of HLA-G. The amino acid sequences of B2M, the linker and the alpha chain of HLA-G contained in the SCD may be sequences comprising sequences having 80% or more, 85% or more, 90% or more, 95% or more, or 97% or more sequence identity with the 1st to 119th amino acid sequence, the 120th to 134th amino acid sequence and the 135th to 448th amino acid sequence of SEQ ID NO: 1 below, respectively.

[SEQ ID NO: 1]
MSRSVALAVLALLSLSGLEAIQRTPKIQVYSRHPAENGKSNFLNCYVSGFHPSDIE
VDLLKNGERIEKVEHSDLSFSKDWSFYLLYYTEFTPTEKDEYACRVNHVTLSQP
KIVKWDRDMGGGGSGGGGSGGGGSGSHSMRYFSAAVSRPGRGEPRFIAMGYV
DDTQFVRFDSDSACPRMEPRAPWVEQEGPEYWEEETRNTKAHAQTDRMNLQT
LRGYYNQSEASSHTLQWMIGCDLGSDGRLLRGYEQYAYDGKDYLALNEDLRS
WTAADTAAQISKRKCEAANVAEQRRAYLEGTCVEWLHRYLENGKEMLQRADP
PKTHVTHHPVFDYEATLRCWALGFYPAEIILTWQRDGEDQTQDVELVETRPAGD
GTFQKWAAVVVPSGEEQRYTCHVQHEGLPEPLMLRWKQSSLPTIPIMGIVAGLV
VLAAVVTGAAVAAVLWRKKSSD

A polynucleotide sequence encoding the amino acid sequence of SEQ ID NO: 1 is set forth in SEQ ID NO: 2. In a preferred embodiment, the polynucleotide sequence encoding the SCD may be a sequence comprising a sequence having 80% or more, 85% or more, 90% or more, 95% or more, or 97% or more sequence identity with the following SEQ ID NO: 2.

In a particularly preferred embodiment, the polynucleotide sequence encoding the SCD is the sequence set forth in SEQ ID NO: 2 below.

[SEQ ID NO: 2]
ATGTCTCGCTCCGTGGCCTTAGCTGTGCTCGCGCTACTCTCTCTTTCTGGCCT
GGAGGCTATCCAGCGTACTCCAAAGATTCAGGTTTACTCACGTCATCCAGCA
GAGAATGGAAAGTCAAATTTCCTGAATTGCTATGTGTCTGGGTTTCATCCATC
CGACATTGAAGTTGACTTACTGAAGAATGGAGAGAGAATTGAAAAAGTGGA
GCATTCAGACTTGTCTTTCAGCAAGGACTGGTCTTTCTATCTCTTGTACTACA
CTGAATTCACCCCCACTGAAAAAGATGAGTATGCCTGCCGTGTGAACCATGT
GACTTTGTCACAGCCCAAGATAGTTAAGTGGGATCGAGACATGGGTGGTGGC
GGATCCGGTGGTGGCGGTTCTGGTGGTGGCGGCTCCGGCTCCCACTCCATGA
GGTATTTCAGCGCCGCCGTGTCCCGGCCCGGCCGCGGGGAGCCCCGCTTCAT
CGCCATGGGCTACGTGGACGACACGCAGTTCGTGCGGTTCGACAGCGACTC

GGCGTGTCCGAGGATGGAGCCGCGGGCGCCGTGGGTGGAGCAGGAGGGGC
CGGAGTATTGGGAAGAGGAGACACGGAACACCAAGGCCCACGCACAGACT
GACAGAATGAACCTGCAGACCCTGCGCGGCTACTACAACCAGAGCGAGGCC
AGTTCTCACACCCTCCAGTGGATGATTGGCTGCGACCTGGGGTCCGACGGAC
GCCTCCTCCGCGGGTATGAACAGTATGCCTACGATGGCAAGGATTACCTCGCC
CTGAACGAGGACCTGCGCTCCTGGACCGCAGCGGACACTGCGGCTCAGATC
TCCAAGCGCAAGTGTGAGGCGGCCAATGTGGCTGAACAAAGGAGAGCCTAC
CTGGAGGGCACGTGCGTGGAGTGGCTCCACAGATACCTGGAGAACGGGAAG
GAGATGCTGCAGCGCGCGGACCCCCCCAAGACACACGTGACCCACCACCCT
GTCTTTGACTATGAGGCCACCCTGAGGTGCTGGGCCCTGGGCTTCTACCCTG
CGGAGATCATACTGACCTGGCAGCGGGATGGGGAGGACCAGACCCAGGACG
TGGAGCTCGTGGAGACCAGGCCTGCAGGGGATGGAACCTTCCAGAAGTGGG
CAGCTGTGGTGGTGCCTTCTGGAGAGGAGCAGAGATACACGTGCCATGTGCA
GCATGAGGGGCTGCCGGAGCCCCTCATGCTGAGATGGAAGCAGTCTTCCCTG
CCCACCATCCCCATCATGGGTATCGTTGCTGGCCTGGTTGTCCTTGCAGCTGT
AGTCACTGGAGCTGCGGTCGCTGCTGTGCTGTGGAGAAAGAAGAGCTCAGA
TTGA

In a preferred embodiment, the linker sequence present between B2M and the alpha chain of HLA-G in the SCD is a peptide composed of glycine and serine (GS linker). The GS linker preferably has a chain length of 15 amino acids or more, and more preferably contains three or more repeats of GGGGS. By using a GS linker having a long chain length, improvement in the expression level of the SCD and improvement in the suppression effect of the SCD on NK cells and macrophages can be obtained.

In a particularly preferred embodiment, the amino acid sequence of the linker sequence present between B2M and the alpha chain of HLA-G in the SCD is the sequence set forth in SEQ ID NO: 3 below.
[SEQ ID NO: 3]
GGGGSGGGGSGGGGS

In another preferred embodiment, the amino acid sequence of the SCD may be a sequence comprising a sequence having 80% or more, 85% or more, 90% or more, 95% or more, or 97% or more sequence identity with the following SEQ ID NO: 4.

In a particularly preferred embodiment, the amino acid sequence of the SCD is the sequence set forth in SEQ ID NO: 4 below. In SEQ ID NO: 4, the 1st to 119th amino acid sequence corresponds to B2M, the 120th to 134th amino acid sequence corresponds to a linker, and the 135th to 477th amino acid sequence corresponds to the alpha chain of HLA-G. The amino acid sequences of B2M, the linker and the alpha chain of HLA-G contained in the SCD may be sequences comprising sequences having 80% or more, 85% or more, 90% or more, 95% or more, or 97% or more sequence identity with the 1st to 119th amino acid sequence, the 120th to 134th amino acid sequence and the 135th to 477th amino acid sequence of SEQ ID NO: 4 below, respectively.

[SEQ ID NO: 4]
MSRSVALAVLALLSLSGLEAIQRTPKIQVYSRHPAENGKSNFLNCYVSGFHPSDIE
VDLLKNGERIEKVEHSDLSFSKDWSFYLLYYTEFTPTEKDEYACRVNHVTLSQP
KIVKWDRDMGGGGSGGGGSGGGGSMKTPRMVVMAPRTLFLLLSGALTLTETW
AGSHSMRYFSAAVSRPQRGEPRFIAMGYVDDTQFVRFDSDSACPRMEPRAPWV
EQEGPEYWEEETRNTKAHAQTDRMNLQTLRGYYNQSEASSHTLQWMIGCDLG
SDGRLLRGYEQYAYDGKDYLALNEDLRSWTAADTAAQISKRKCEAANVAEQR
RAYLEGTCVEWIHRYLENGKEMLQRADPPKTHVTHHPVFDYEATLRCWALGFY
PAEIILTWQRDGEDQTQDVELVETRPAGDGTFQKWAAVVVPSGEEQRYTCHVQ
HEGLPEPLMLRWKQSSLPTIPIMGIVAGLVVLAAVVTGAAVAAVIWRKKSSD

A polynucleotide sequence encoding the amino acid sequence of SEQ ID NO: 4 is set forth in SEQ ID NO: 5.

In a preferred embodiment, the polynucleotide sequence encoding the SCD may be a sequence comprising a sequence having 80% or more, 85% or more, 90% or more, 95% or more, or 97% or more sequence identity with the following SEQ ID NO: 5.

In a particularly preferred embodiment, the polynucleotide sequence encoding the SCD is the sequence set forth in SEQ ID NO: 5 below.

[SEQ ID NO: 5]
ATGTCTCGCTCCGTGGCCTTAGCTGTGCTCGCGCTACTCTCTCTTTCTGGCCT
GGAGGCTATCCAGCGTACTCCAAAGATTCAGGTTTACTCACGTCATCCAGCA
GAGAATGGAAAGTCAAATTTCCTGAATTGCTATGTGTCTGGGTTTCATCCATC
CGACATTGAAGTTGACTTACTGAAGAATGGAGAGAGAATTGAAAAAGTGGA
GCATTCAGACTTGTCTTTCAGCAAGGACTGGTCTTTCTATCTCTTGTACTACA
CTGAATTCACCCCCACTGAAAAAGATGAGTATGCCTGCCGTGTGAACCATGT
GACTTTGTCACAGCCCAAGATAGTTAAGTGGGATCGAGACATGGGTGGTGGC
GGATCCGGTGGTGGCGGTTCTGGTGGTGGCGGCTCCATGAAGACGCCAAGG
ATGGTGGTCATGGCGCCCCGAACCCTCTTCCTGCTGCTCTCGGGGGCCCTGA
CCCTGACCGAGACCTGGGCGGGCTCCCACTCCATGAGGTATTTCAGCGCCGC
CGTGTCCCGGCCCGGCCGCGGGGAGCCCCGCTTCATCGCCATGGGCTACGTG
GACGACACGCAGTTCGTGCGGTTCGACAGCGACTCGGCGTGTCCGAGGATG
GAGCCGCGGGCGCCGTGGGTGGAGCAGGAGGGGCCGGAGTATTGGGAAGA
GGAGACACGGAACACCAAGGCCCACGCACAGACTGACAGAATGAACCTGC
AGACCCTGCGCGGCTACTACAACCAGAGCGAGGCCAGTTCTCACACCCTCC
AGTGGATGATTGGCTGCGACCTGGGGTCCGACGGACGCCTCCTCCGCGGGTA
TGAACAGTATGCCTACGATGGCAAGGATTACCTCGCCCTGAACGAGGACCTG
CGCTCCTGGACCGCAGCGGACACTGCGGCTCAGATCTCCAAGCGCAAGTGT
GAGGCGGCCAATGTGGCTGAACAAAGGAGAGCCTACCTGGAGGGCACGTGC
GTGGAGTGGCTCCACAGATACCTGGAGAACGGGAAGGAGATGCTGCAGCGC
GCGGACCCCCCCAAGACACACGTGACCCACCACCCTGTCTTTGACTATGAGG
CCACCCTGAGGTGCTGGGCCCTGGGCTTCTACCCTGCGGAGATCATACTGAC
CTGGCAGCGGGATGGGGAGGACCAGACCCAGGACGTGGAGCTCGTGGAGA
CCAGGCCTGCAGGGGATGGAACCTTCCAGAAGTGGGCAGCTGTGGTGGTGC
CTTCTGGAGAGGAGCAGAGATACACGTGCCATGTGCAGCATGAGGGGCTGC
CGGAGCCCCTCATGCTGAGATGGAAGCAGTCTTCCCTGCCCACCATCCCCAT
CATGGGTATCGTTGCTGGCCTGGTTGTCCTTGCAGCTGTAGTCACTGGAGCTG

CGGTCGCTGCTGTGCTGTGGAGAAAGAAGAGCTCAGATTGA

### [Single-chain fusion peptide mimicking HLA-E (SCT)]

The single-chain fusion peptide mimicking HLA-E is a single-chain trimer (SCT) fusion peptide comprising "signal peptide of HLA-A, HLA-B, HLA-C or HLA-G," "B2M" and "alpha chain of HLA-E."

The "signal peptide of HLA-A, HLA-B, HLA-C or HLA-G" (hereinafter, also referred to as "signal peptide") is an endogenous peptide presented on HLA-E.

In the SCT, it is preferable that "signal peptide," "B2M" and "alpha chain of HLA-E" are present in this order from the N-terminal side toward the C-terminal side.

In the present invention, a known amino acid sequence of a signal peptide can be used. As the signal peptide, a signal peptide of HLA-G is preferred. The amino acid sequence of the signal peptide is preferably a wild-type sequence, but one or a plurality of amino acids may be deleted, substituted or added as long as the function of the SCT is not impaired.

In the present invention, a known amino acid sequence of B2M can be used. The amino acid sequence of B2M is preferably a wild-type sequence, but one or a plurality of amino acids may be deleted, substituted or added as long as the function of the SCT is not impaired.

In the present invention, a known amino acid sequence of the alpha chain of HLA-E can be used. The amino acid sequence of the alpha chain of HLA-E is preferably a wild-type sequence, but one or a plurality of amino acids may be deleted, substituted or added as long as the function of the SCT is not impaired.

In a preferred embodiment, the amino acid sequence of the SCT may be a sequence comprising a sequence having 80% or more, 85% or more, 90% or more, 95% or more, or 97% or more sequence identity with the following SEQ ID NO: 6.

In a particularly preferred embodiment, the amino acid sequence of the SCT is the sequence set forth in SEQ ID NO: 6 below. In SEQ ID NO: 6, the 1st to 20th amino acid sequence corresponds to the N-terminal 20 amino acids of B2M, the 21st to 29th amino acid sequence corresponds to the signal peptide of HLA-G, the 30th to 44th amino acid sequence corresponds to a linker (first linker), the 45th to 143rd amino acid sequence corresponds to the 99 amino acid sequence on the C-terminal side of B2M, the 144th to 163rd amino acid sequence corresponds to a linker (second linker), and the 164th to 500th amino acid sequence corresponds to the alpha chain of HLA-E. The amino acid sequences of the signal peptide of HLA-G, the first linker, B2M, the second linker and the alpha chain of HLA-E contained in the SCT may be sequences having 80% or more, 85% or more, 90% or more, 95% or more, or 97% or more sequence identity with the 21st to 29th amino acid sequence, the 30th to 44th amino acid sequence, the sequence combining the 1st to 20th amino acid sequence and the 45th to 143rd amino acid sequence, the 144th to 163rd amino acid sequence and the 164th to 500th amino acid sequence of SEQ ID NO: 6 below, respectively.

[SEQ ID NO: 6]
MSRSVALAVLALLSLSGLEAVMAPRTLFLGGGGSGGGGSGGGGSIQRTPKIQVY
SRHPAENGKSNFLNCYVSGFHPSDIEVDLLKNGERIEKVEHSDLSFSKDWSFYLL
YYTEFTPTEKDEYACRVNHVTLSQPKIVKWDRDMGGGGSGGGGSGGGGSGGG
GSGSHSLKYFHTSVSRPGRGEPRFISVGYVDDTQFVRFDNDAASPRMVPRAPW
MEQEGSEYWDRETRSARDTAQIFRVNLRTLRGYYNQSEAGSHTLQWMHGCEL
GPDGRFLRGYEQFAYDGKDYLTLNEDLRSWTAVDTAAQISEQKSNDASEAEHQ
RAYLEDTCVEWLHKYLEKGKETLLHLEPPKTHVTHHPISDHEATLRCWALGFYP
AEITLTWQQDGEGHTQDTELVETRPAGDGTFQKWAAVVVPSGEEQRYTCHVQH
EGLPEPVTLRWKPASQPTIPIVGIIAGLVLLGSVVSGAVVAAVIWRKKSSGGKGGS
YSKAEWSDSAQGSESHSL

A polynucleotide sequence encoding the amino acid sequence of SEQ ID NO: 6 is set forth in SEQ ID NO: 7.

In a preferred embodiment, the polynucleotide sequence encoding the SCT may be a sequence comprising a sequence having 80% or more, 85% or more, 90% or more, 95% or more, or 97% or more sequence identity with the following SEQ ID NO: 7.

In a particularly preferred embodiment, the polynucleotide sequence encoding the SCT is the sequence set forth in SEQ ID NO: 7 below.

[SEQ ID NO: 7]
ATGTCTCGCTCCGTGGCCTTAGCTGTGCTCGCGCTACTCTCTCTTTCTGGCCT
GGAGGCTGTCATGGCGCCCCGAACCCTCTTCCTGGGTGGTGGCGGATCCGGT
GGTGGCGGTTCTGGTGGTGGCGGCTCCATCCAGCGTACTCCAAAGATTCAGG
TTTACTCACGTCATCCAGCAGAGAATGGAAAGTCAAATTTCCTGAATTGCTAT
GTGTCTGGGTTTCATCCATCCGACATTGAAGTTGACTTACTGAAGAATGGAG
AGAGAATTGAAAAAGTGGAGCATTCAGACTTGTCTTTCAGCAAGGACTGGT
CTTTCTATCTCTTGTACTACACTGAATTCACCCCCACTGAAAAAGATGAGTAT
GCCTGCCGTGTGAACCATGTGACTTTGTCACAGCCCAAGATAGTTAAGTGGG
ATCGAGACATGGGTGGTGGCGGTTCTGGTGGTGGCGGTAGTGGCGGCGGAG
GAAGCGGTGGTGGCGGTTCCGGCTCCCACTCCTTGAAGTATTTCCACACTTC
CGTGTCCCGGCCCGGCCGCGGGGAGCCCCGCTTCATCTCTGTGGGCTACGTG
GACGACACCCAGTTCGTGCGCTTCGACAACGACGCCGCGAGTCCGAGGATG
GTGCCGCGGGCGCCGTGGATGGAGCAGGAGGGGTCAGAGTATTGGGACCGG
GAGACACGGAGCGCCAGGGACACCGCACAGATTTTCCGAGTGAATCTGCGG
ACGCTGCGCGGCTACTACAATCAGAGCGAGGCCGGGTCTCACACCCTGCAGT
GGATGCATGGCTGCGAGCTGGGGCCCGACGGGCGCTTCCTCCGCGGGTATGA
ACAGTTCGCCTACGACGGCAAGGATTATCTCACCCTGAATGAGGACCTGCGC
TCCTGGACCGCGGTGGACACGGCGGCTCAGATCTCCGAGCAAAAGTCAAAT
GATGCCTCTGAGGCGGAGCACCAGAGAGCCTACCTGGAAGACACATGCGTG
GAGTGGCTCCACAAATACCTGGAGAAGGGGAAGGAGACGCTGCTTCACCTG
GAGCCCCCAAAGACACACGTGACTCACCACCCCATCTCTGACCATGAGGCC
ACCCTGAGGTGCTGGGCCCTGGGCTTCTACCCTGCGGAGATCACACTGACCT
GGCAGCAGGATGGGGAGGGCCATACCCAGGACACGGAGCTCGTGGAGACCA
GGCCTGCAGGGGATGGAACCTTCCAGAAGTGGGCAGCTGTGGTGGTGCCTT
CTGGAGAGGAGCAGAGATACACGTGCCATGTGCAGCATGAGGGGCTACCCG
AGCCCGTCACCCTGAGATGGAAGCCGGCTTCCCAGCCCACCATCCCCATCGT
GGGCATCATTGCTGGCCTGGTTCTCCTTGGATCTGTGGTCTCTGGAGCTGTGG

TTGCTGCTGTGATATGGAGGAAGAAGAGCTCAGGTGGAAAAGGAGGGAGCT
ACTCTAAGGCTGAGTGGAGCGACAGTGCCCAGGGGTCTGAGTCTCACAGCT
TGTAA

In a preferred embodiment, the linker sequence present between the signal peptide and B2M in the SCT is a peptide composed of glycine and serine (GS linker). The GS linker preferably has a chain length of 15 amino acids or more, and more preferably contains three or more repeats of GGGGS. By using a GS linker having a long chain length, improvement in the expression level of the SCT and improvement in the suppression effect of the SCT on NK cells and macrophages can be obtained.

In a particularly preferred embodiment, the amino acid sequence of the linker sequence present between the signal peptide and B2M in the SCT is the sequence set forth in SEQ ID NO: 8 below.
[SEQ ID NO: 8]
GGGGSGGGGSGGGGS

In a preferred embodiment, the linker sequence present between B2M and the alpha chain of HLA-E in the SCT is a peptide composed of glycine and serine (GS linker). The GS linker of this embodiment preferably has a chain length of 15 amino acids or more, and more preferably contains three or more repeats of GGGS. By using a GS linker having a long chain length, improvement in the expression level of the SCT and improvement in the suppression effect of the SCT on NK cells and macrophages can be obtained. Also, in a particularly preferred embodiment, the amino acid sequence of the linker sequence present between B2M and the alpha chain of HLA-E in the SCT is the sequence set forth in SEQ ID NO: 9 below.
[SEQ ID NO: 9]
GGGGSGGGGSGGGGS

In the present invention, at least one of the SCD or the SCT is knocked into, and preferably both the SCD and the SCT are knocked in.

### [Knock-in of polynucleotide encoding SCD and/or SCT]

In the present specification, when specifying a position in a genome, a position in the hg38 genome sequence as a reference genome is used.

hg38 is a reference genome released in December 2013 by the University of California, Santa Cruz (UCSC). A reference genome is a genome for reference created by combining various genomes, and it does not mean that a human having this genome exists. However, by checking fragmentary sequence information decoded from genomic DNA of a human individual against the reference genome, the decoded fragmentary sequence information is connected to construct a continuous sequence on a computer, whereby the sequence of the genomic DNA of the human individual can be estimated. Thus, decoding genomic DNA of an individual such as a human individual is usually performed by associating the sequence of the genomic DNA of the human individual with the reference genome.

A position or region corresponding to a specific position or specific region of the hg38 genome sequence means a position or region linked to the specific position or specific region in a genome of another individual having a different specific sequence. Specifically, a position or region having a sequence characteristic of the position or region based on sequence identity is a position or region corresponding to the specific position or specific region of the hg38 genome sequence. The corresponding position can be determined by alignment of subsequences of two genomic DNAs. Even if there is a difference in specific sequences, the correspondence relationship between two genomic DNAs can be determined by performing alignment if they have an ortholog relationship or have sequence identity.

In a region rich in paralogs generated by gene duplication, simply determining the correspondence relationship of sequences based on individual sequences may not be sufficient to determine the true correspondence relationship between two genomes. When determining the corresponding sequence, the correspondence relationship between two genomes can be clarified by requiring high sequence identity. Further, when the specific region is a large region containing a plurality of genes, synteny can be considered. Synteny means that the physical positional relationship of orthologs on the genome is conserved. Synteny can exist between individuals and between organisms. Therefore, the specific region can be determined in consideration of synteny.

In one aspect of the present invention, the "region containing alpha chain genes of HLA-F, HLA-G and HLA-A" of the cell is replaced (knocked in) by a "polynucleotide encoding SCD (hereinafter, also referred to as "SCD coding sequence")."

The "region containing alpha chain genes of HLA-F, HLA-G and HLA-A" includes a "region corresponding to an HLA-F alpha chain gene region on chr6 of the hg38 genome sequence," a "region corresponding to an HLA-G alpha chain gene region on chr6 of the hg38 genome sequence," and a "region corresponding to an HLA-A alpha chain gene region on chr6 of the hg38 genome sequence" shown below.

| | Position on chr6 of hg38 genome sequence |
|---|---|
| HLA-F alpha chain | 29,723,657-29,726,953 |
| HLA-G alpha chain | 29,828,046-29,829,919 |
| HLA-A alpha chain | 29,942,756-29,945,281 |

The "region containing alpha chain genes of HLA-F, HLA-G and HLA-A" may contain an upstream region of the HLA-F alpha chain gene (a region corresponding to a region upstream of 29,723,657 on chr6 of the hg38 genome sequence) and/or a downstream region of the HLA-A alpha chain gene (a region corresponding to a region downstream of 29,945,281 on chr6 of the hg38 genome sequence).

The length of the upstream region of the HLA-F alpha chain gene is not particularly limited as long as a knock-in method of the "SCD coding sequence" described later can be carried out, but is, for example, 800 to 20,000 nucleotides, preferably 1,000 to 5,000 nucleotides. The length of the upstream region can be appropriately set in consideration of the design position of the guide RNA, the fact that the homology arm is a specific sequence, and the like.

The length of the downstream region of the HLA-A alpha chain gene is not particularly limited as long as a knock-in method of the "SCD coding sequence" described later can be carried out, but is, for example, 800 to 20,000 nucleotides, preferably 1,000 to 5,000 nucleotides. The length of the downstream region can be appropriately set in consideration of the design position of the guide RNA, the fact that the homology arm is a specific sequence, and the like.

In a preferred embodiment of the present invention, the "region containing alpha chain genes of HLA-F, HLA-G and HLA-A" is a chromosomal region corresponding to a chromosomal region of chr6: 29,704,906-29,958,642 of the hg38 genome sequence (target region 1).

In another preferred embodiment of the present invention, the "region containing alpha chain genes of HLA-F, HLA-G and HLA-A" is a chromosomal region corresponding to a chromosomal region of chr6: 29,707,019-29,956,470 of the hg38 genome sequence (target region 2).

In another aspect of the present invention, the "region containing alpha chain genes of HLA-C and HLA-B" of the cell is replaced (knocked in) by a "polynucleotide encoding SCT (hereinafter, also referred to as "SCT coding sequence")."

The "region containing alpha chain genes of HLA-C and HLA-B" includes a "region corresponding to an HLA-C alpha chain gene region on chr6 of the hg38 genome sequence" and a "region corresponding to an HLA-B alpha chain gene region on chr6 of the hg38 genome sequence" shown below.

| | Position on chr6 of hg38 genome sequence |
|---|---|
| HLA-C alpha chain | 31,269,338-31,271,868 |
| HLA-B alpha chain | 31,354,486-31,356,958 |

The "region containing alpha chain genes of HLA-C and HLA-B" may contain an upstream region of the HLA-C alpha chain gene (a region corresponding to a region upstream of 31,269,338 on chr6 of the hg38 genome sequence) and/or a downstream region of the HLA-B alpha chain gene (a region corresponding to a region downstream of 31,356,958 on chr6 of the hg38 genome sequence).

The length of the upstream region of the HLA-C alpha chain gene is not particularly limited as long as a knock-in method of the "SCT coding sequence" described later can be carried out, but is, for example, 800 to 60,000 nucleotides, preferably 1,000 to 5,000 nucleotides. The length of the upstream region can be appropriately set in consideration of the design position of the guide RNA, the fact that the homology arm is a specific sequence, and the like.

The length of the downstream region of the HLA-B alpha chain gene is not particularly limited as long as a knock-in method of the "SCT coding sequence" described later can be carried out, but is, for example, 800 to 35,000 nucleotides, preferably 1,000 to 5,000 nucleotides. The length of the downstream region can be appropriately set in consideration of the design position of the guide RNA, the fact that the homology arm is a specific sequence, and the like.

In a preferred embodiment of the present invention, the "region containing alpha chain genes of HLA-C and HLA-B" is a chromosomal region corresponding to a chromosomal region of chr6: 31,224,837-31,378,196 of the hg38 genome sequence.

In another preferred embodiment of the present invention, the "region containing alpha chain genes of HLA-C and HLA-B" is a chromosomal region corresponding to a chromosomal region of chr6: 31,227,286-31,378,780 of the hg38 genome sequence.

In a preferred embodiment of the present invention, in the cell,
the "region containing alpha chain genes of HLA-F, HLA-G and HLA-A" is replaced (knocked in) by the "SCD coding sequence," and
the "region containing alpha chain genes of HLA-C and HLA-B" is replaced (knocked in) by the "SCT coding sequence."

These gene modifications can be carried out using a gene modification method utilizing a site-specific nuclease (for example, methods described in eLife, 2017, 6, e27873, FEBS Letters, 591, 2017, 903-913 and International Publication No. WO 2021/206054).

Hereinafter, a method for modifying genes of a diploid cell having two alleles using a CRISPR/Cas system as a site-specific nuclease will be described.

Note that the gene modification method can also be carried out using a site-specific nuclease other than the CRISPR/Cas system (for example, TALEN, ZFN, etc.).

### 1. Method for knocking in SCD

### [Step 1] Substitution of region containing alpha chain genes of HLA-F, HLA-G and HLA-A with selection marker gene

The "region containing alpha chain genes of HLA-F, HLA-G and HLA-A" (hereinafter, also referred to as "target region") replaced in this step is, for example, a chromosomal region corresponding to chr6: 29,704,906-29,958,642 (target region 1) of the hg38 genome sequence.

### [1-1] Site-specific cleavage of DNA using CRISPR/Cas system

When the target region is the target region 1, two double-strand breaks per allele are introduced by a CRISPR/Cas9 system using a guide RNA (gRNA) targeting an upstream region of the HLA-F alpha chain gene (chr6: 29,707,002-29,707,024 of hg38 genome sequence), a gRNA targeting a downstream region of the HLA-A alpha chain gene (chr6: 29,956,465-29,956,487 of hg38 genome sequence), and Cas9 protein.

The sequence of the gRNA does not have to be completely identical to the target sequence as long as it can bind to the target sequence. For introduction of the gRNA, a polynucleotide encoding the gRNA (for example, an expression vector) may be used.

A method for introducing the gRNA and Cas9 into cells is not particularly limited, and a known method (for example, lipofection method) can be used without particular limitation.

Note that step [1-1] may be carried out using another site-specific nuclease, for example, TALEN, ZFN, or the like, instead of the CRISPR/Cas system.

### [1-2] Introduction of different selection marker genes into target region of each allele

In order to introduce the selection marker gene, a donor DNA for selection marker is used.

The "donor DNA for selection marker" contains a "selection marker gene" between an "upstream homology arm sequence" and a "downstream homology arm sequence."

The "upstream homology arm sequence" has a sequence capable of homologous recombination with an upstream sequence in the target region (for example, chr6: 29,704,906-29,707,018 of hg38 genome sequence when the target region is the target region 1) present in one of the two chromosomes (chromosome to be subjected to homologous recombination). The upstream homology arm sequence does not have to be completely identical to the upstream sequence in the target region as long as homologous recombination is possible, but preferably has 90% or more, more preferably 95% or more sequence identity.

The "downstream homology arm sequence" has a sequence capable of homologous recombination with a downstream sequence in the target region (for example, chr6: 29,956,471-29,958,642 of hg38 genome sequence when the target region is the target region 1) present in one of the two chromosomes (chromosome to be subjected to homologous recombination). The downstream homology arm sequence does not have to be completely identical to the downstream sequence in the target region as long as homologous recombination is possible, but preferably has 90% or more, more preferably 95% or more sequence identity.

The lengths of the upstream homology arm and the downstream homology arm can be, for example, about 500 bp to 3 kbp, but can be appropriately designed by those skilled in the art in the same manner as in the design of ordinary donor DNA.

The "donor DNA for selection marker" contains a positive selection marker (for example, drug resistance gene) used in this step and a negative selection marker (for example, fluorescent protein) used in Steps 2 and 3 described later. The positive selection marker allows selection of target cells based on its expression. The negative selection marker allows selection of target cells based on the absence of its expression. The selection marker can be appropriately selected according to the type of cells to be used and the like.

The selection marker may be one that serves as both a positive selection marker and a negative selection marker (dual-purpose marker). Examples of the dual-purpose marker include fluorescent proteins.

In this step, two types of donor DNAs for selection marker are used. The two types of donor DNAs for selection marker each have a mutually distinguishably different positive selection marker gene. "Mutually distinguishably different" means that they can be distinguished from each other in physiological properties (for example, drug resistance) and the like imparted to cells by the positive selection marker gene.

For example, when two types of drug resistance genes showing resistance to different types of drugs are used as the positive selection marker genes, only cells in which the target regions of the two alleles have been replaced with the different drug resistance genes show resistance to the two types of drugs.

Furthermore, the two types of donor DNAs for selection marker each have a mutually distinguishably different negative selection marker gene.

Examples of combinations of selection marker genes possessed by the two types of donor DNAs for selection marker are shown below.

| | Positive selection marker | Negative selection marker |
|---|---|---|
| One donor DNA for selection marker | Puromycin resistance gene | Green fluorescent protein |
| Other donor DNA for selection marker | Blasticidin resistance gene | Red fluorescent protein |

The donor DNA for selection marker may be either linear or circular, but is preferably circular. Preferably, the donor DNA for selection marker is a plasmid. The donor DNA for selection marker may contain a spacer sequence between the above sequences.

A method for introducing the donor DNA for selection marker into cells is not particularly limited, and a known method (for example, lipofection method) can be used without particular limitation.

It is preferable to simultaneously introduce the gRNA and Cas9 and the donor DNA for selection marker into cells to carry out step [1-1] and step [1-2] simultaneously.

Homologous recombination (HDR) occurs between the donor DNA for selection marker having the "upstream homology arm sequence" and the "downstream homology arm sequence" and the chromosome of the cell. Substitution of sequences by homologous recombination can be carried out using well-known and commonly used techniques. After homologous recombination, cells expressing the two types of positive selection markers are selected and used in Step 2.

In the selected cells, the target regions of the two alleles are replaced with different types of selection marker genes, respectively. In other words, in the selected cells, the alpha chain genes of HLA-F, HLA-G and HLA-A of the two alleles are knocked out, and instead, different types of selection marker genes are knocked into each allele.

### [Step 2] Substitution of selection marker gene with SCD-encoding polynucleotide in one allele

### [2-1] Site-specific cleavage of DNA using CRISPR/Cas system in one allele

One double-strand break per allele is introduced by a CRISPR/Cas9 system using a gRNA targeting one of the two types of positive selection marker genes used in step [1-2] and Cas9 protein.

The sequence of the gRNA does not have to be completely identical to the target sequence as long as it can bind to the target sequence. For introduction of the gRNA, a polynucleotide encoding the gRNA (for example, an expression vector) may be used.

A method for introducing the gRNA and Cas9 into cells is not particularly limited, and a known method (for example, lipofection method) can be used without particular limitation.

Note that step [2-1] may be carried out using another site-specific nuclease, for example, TALEN, ZFN, or the like, instead of the CRISPR/Cas system.

### [2-2] Introduction of SCD-encoding polynucleotide into only one allele

In order to introduce the SCD-encoding polynucleotide (hereinafter, also referred to as "SCD coding sequence"), a donor DNA for SCD is used.

The "donor DNA for SCD" contains the "SCD coding sequence" (for example, SEQ ID NO: 2) between an "upstream homology arm sequence" and a "downstream homology arm sequence."

The "upstream homology arm sequence" and the "downstream homology arm sequence" of the donor DNA for SCD are the same as the "upstream homology arm sequence" and the "downstream homology arm sequence" of the donor DNA for selection marker used in step [1-2], respectively. Therefore, homologous recombination occurs between the "donor DNA for SCD" and the "allele containing the selection marker gene," and the selection marker gene is replaced by the SCD coding sequence. Substitution of sequences by homologous recombination can be carried out using well-known and commonly used techniques.

Note that as long as homologous recombination occurs, the "upstream homology arm sequence" and the "downstream homology arm sequence" of the donor DNA for SCD may be different from the "upstream homology arm sequence" and the "downstream homology arm sequence" of the donor DNA for selection marker used in step [1-2], respectively.

For the purpose of increasing the expression level of the SCD, the donor DNA for SCD preferably contains a promoter sequence of an alpha chain gene of HLA-A (preferably a sequence corresponding to chr6: 29,938,509-29,942,553 of hg38 genome sequence) upstream of the SCD coding sequence. Here, since what SCD mimics is HLA-G, use of a promoter of an alpha chain gene of HLA-G is conceivable. However, HLA-G is known to be expressed in limited cells (placental cells, etc.). On the other hand, HLA-A is known to be stably expressed in multiple cell lineages. When applying the present invention to human pluripotent stem cells, since it is assumed that the obtained gene-modified cells are transplanted after being differentiated into various cell lineages, it is preferable to use the promoter sequence of the alpha chain gene of HLA-A.

In addition, by using the promoter sequence of the alpha chain gene of HLA-A which has inducibility by interferon gamma stimulation, it is also possible to control the timing of expressing the SCD.

In the present invention, a known promoter sequence of an alpha chain gene of HLA-A can be used. In a preferred embodiment, the promoter sequence of the alpha chain gene of HLA-A may be a sequence comprising a sequence having 80% or more, 85% or more, 90% or more, 95% or more, or 97% or more sequence identity with the following SEQ ID NO: 10.

In a particularly preferred embodiment, the promoter sequence of the alpha chain gene of HLA-A is the sequence set forth in SEQ ID NO: 10 below.

[SEQ ID NO: 10]
AAGACAAGCTTAAAGAGTTAATGAAAAATAATTAGATACAAGAAGACTTTGA
TTTTCAGAAACCTGAAACAATAGTTATAATTTTGCTTTTAACGTATATTCAAAT
CCTTTGATACTGTTCCTTTCTAGAGGTGCAGCTTAATTCCCTCTCTTGAGTGTG
GCTTGGACTTAATGAGGCACTTCTGAAATGGCCTGGTTCTGTGTTCCCACCCA
AATCTCATCTTGAATTGTTATGCAAATTGTAATCCCTACCTATTGGGGGAGGGA
CCTCATGGGAGGTGATTGGATCATGGGGACGGTGCCCCCATGCTGTTCTCCTG
ATGCTGAGGGAATTCTCATGAGATCTGATGGTTTTATAAGGGGCTTTTCCCTG
CTTCATTGTGCATTTCTCTCTCCTGTCACCACGTGAAGAAGGACGGGTTTGCT
TCCACTTCTGCCGTGACTGTAAGTTTCCTGGGGCAGCCTCCTCAGTCATGCA
GAACTGTGAGTCAATTAAATCTCTTTCCTTTATAAATTACCCAGTCTCATGTAT
TTCTTTATAGCAGTGTGAGAATGGACTAATACAACTTCTAACTTATAGAATAGT
GCCAACATAACAGTTTGTGACTCTGGGTGTAGAACATAAAACTAACTGCGGC
TTCCACCTTCTCTCTCTCTGAATCTGGGATCATGAGCTCTGGGGGAAGCCAGC
CGCTCTGCCATAAGCAGCCCTGCAGGAAGGTCCACATGACTGAGAACTGAG
GCCTTCTGGGAACAGACAACAAGGAACCAGGCCTTTTCCAACAGCCATGTG
ACTGATCCATGTTTCTTGTGAATTCCCAGCCCCAGCGAAGCCCTTGGATGCTG
CGGCCCCTGGCTGACAACTGGAGTGCAGCCTTTTGAGAGGCCCTGAGCAGG
AAGCACTCAGGGAAACCTTTCCTGGATTCCTGACGATTGGAAACTGTGGGAG
ATGAGAAACATTTGTTGTTTCGAGCTAAGTTTTACGTAATTTGTTATGCAATAG

TAAATAATATATTTTCACAAGAGAAGATGTATTATTACACATCAAATTGCATTT
GCTCTAAATGTGTCATCATCATCATTATTATTTTTGAGACAGGGTCTTGCTCTG
TCACCCAGGCTGGAATGCAGTGGCATGATCACCATGCACTGCAGTGTCGAAC
GCCTGGGGTCAAGGGACTCTCTGACCTCAGCCTCCTGAGTAGCTGCGACTAC
CATCATGAACTACCATGCCTGGCTAATTTTCTAATTTTTTGTAGAGATGGAGGT
TTTGCCCAGGTTGATCTTGAACTTCTGGAGTCAACAAATCTCCCTTACTCCAC
CTTCCACAGTGCTATGATGACAGGCGTGAGCCACCATACCTGGCCTAAATTAA
TTATAAGATATTAAACATGTAACTTAGTTTTAAAAGGTAAGGACAATTTCCATG
GCTGAAGAGGATGTATTTTATGACCGTTCACAATGATCACTTTACTTGAACTT
CACTTTCCAACTGTGTCCCAATTAAACACAAAAGGAAGATCCAACCCTTGCT
AGGCTGATTCTATGAAGGCCTCAACAAGCAGCTCCTGGTCATTCACCTTCCTC
CAGTTATTCAACCAACTCTAATATAGGTGCTGCTGTGAAGGGATTTAGCAGAT
ATAATTAAGGGTCTCAATTAGTTGATTTATGCTGGGTTTATCCTGCTTGAACTG
TCCTAATCAGGTGAGCCCTTGAAAGGACTGGGTTCTTCATGAGCATAGAGAC
TTACAGTGTGAGAGGGACTCAGCATGAGGGGTTTCCTCCACCATGGGCTTTG
AAAAGGAAAGGGCTATGGGCCGGGCGCGGTGGCTCACGCCTGTAATCCCGA
CACTTTGGGAGGCCGAGGCGGGTGGATCATGAGGTCAGGAGGTTGAGACAA
TCCTGGCTAACAAGGTGAAACCCTGTCTCTACTAAGAAAAAAAAAAAAAATT
AGCAGAGTGTAGTGGTGGGCACCTGTAGTCTCAGCTACTTGGGACTGAGACA
GGAGAATGGTGTGAACCCAGGAGGTGGAGCTTGTAGTGAGCAGAGATCATT
GGGCCACTGTACCCCAGCCTGGGCTACAGAGCCAGACTCCGTCTCAAAAAA
AAAAAAAAAAAAAAAAAAAAAATTAAGGGGCTGTGTAGGAAAGAATGCTG
GTGAGCACCGGGAATTGAGCCCCTCCCAGTTCTCTACACTGACAGCTAGCCA
GGAACAGGGACCTCAGTCTTTACAACTGCCAAAAACTGCATTCCGCCACCTC
TGTATAAACCTGAAGGAGGATTCAAAATGAAAACACAGCTTTTGGAAGCCCA
GAACAGAGATTCTATCCACATCTTGCCCAGATTTCTGACCAAGGAACTATAAG
CAGATAAATGGGTGTTGTTTCGCCAGGCGTGGTAGTGTGCGAATGAATTGATA
TACACACTAGTTGCATAAAATAAAATCTTTCTGAACTTTTTCAGTGTTTTACAG
TTTATAATTATCTGTGATGCAATTTAATACACTCATATTTCATTCATTAAATCAA
CAAAAATTAACTTAGTCCCTACAATGAACCAGGTATCCCCTCATATGCTCAAG

TGCCTGACACTCCAGAAGCTTCACAAGACCGAGGTGGAGACACTGGAGTGT
TTTAAGTGGAGAAATAACACACTCCGACTCACAGGAGCAGGACCACTGTGA
AAAAAACAGTTACGTAGCAGGTCATGGGACAGTGCTAGTGTCACAATTCATG
AGTGACAGTGTGGTGGGGACTAAGGGGACAGGAGGGCCTGAAGGATGAGA
AGGACGGAGGGAAGGGCTGGAGAAGCAGGAGGTGAGGAAAAGGAGCAGA
GGAAAGAATTTGAAAGCAGCAGAATTCTTAGGTTTAAATACATTGTTTTATGG
ATTTTAATACATCCATCTACAGAGCCTAGCAGGGTGTCCTTGGCAGTTGTCTT
TTAATACCTCATGTGGGTCTGCCTAAAAACTAATTTTTTATGTTAATCAGGTTT
AAAAAATACTAAGTGTTCCTATAAAATATACACAACACTTAGAAGTGGATACT
TCCTAAAAACAGACAGTGCATGAGCACTAGTGAGGGGCATTGTGAGTGCATT
GAACAGTTGCAACTTTGAGGTGAATAAAGCCTGTAATGGCTTCTGGTTGCAA
CATATAGGAACACAGTCGCTACTTTGTATTGAGGAGATGTCCTGGACTCACAC
AGAAACTCAGAGCTATGGAATGATGGTAAATTTAAAATACTACAAGCAGGAG
TCACAGATACATTGTCTGGGAAAATGCAACTTAGTAGCTTTGTGAGTCCTGTT
GTAAGGCTTTTGGACACATTTATACATCAAGGGGCCAAAGTCACATTTTTTAC
CTATTAGATTCCTGATCATTCAGGGGTTACCAAGATTCTGCTACCCACTGTAGT
TAATAAACAAAGAGCAAATTGGTCTCTATTCTGTCTCATGCACTCAGGCGCAA
CTCTTCCCGATTAAAAACAAAAACAACAACAACAAAAATCTACACCTCCATT
CCCAGAGCAAGCTTACTCTCTGGCACCAAACTCCATGGGATGATTTTTCTTCT
AGAAGAGTCCAGGTGGACAGGTAAGGAGTGGGAGTCAGGGAGTCCAGTTC
AGGGACAGAGATTACGGGATAAAAAGTGAAAGGAGAGGGACGGGGCCCAT
GCCGAGGGTTTCTCCCTTGTTTCTCAGACAGCTCTTGGGCCAAGACTCAGGG
AGACATTGAGACAGAGCGCTTGGCACAGAAGCAGAGGGGTCAGGGCGAAG
TCCCAGGGCCCCAGGCGTGGCTCTCAGGGTCTCAGGCCCCGAAGGCGGTGT
ATGGATTGGGGAGTCCCAGCCTTGGGGATTCCCCAACTCCGCAGTTTCTTTTC
TCCCTCTCCCAACCTATGTAGGGTCCTTCTTCCTGGATACTCACGACGCGGAC
CCAGTTCTCACTCCCATTGGGTGTCGGGTTTCCAGAGAAGCCAATCAGTGTC
GTCGCGGTCGCGGTTCTAAAGTCCGCACGCACCCACCGGGACTCAGATTCTC
CCCAGACGCCGAGG

The promoter sequence of the alpha chain gene of HLA-A is knocked in in a manner operably linked to the SCD coding sequence. "Operably linked" means that the promoter sequence of the alpha chain gene of HLA-A is arranged relative to the SCD coding sequence in a manner that controls the expression of the SCD coding sequence.

In another aspect of the present invention, a promoter sequence of a gene encoding an alpha chain of an HLA Class I molecule other than HLA-G may be knocked in in a manner operably linked to the SCD coding sequence.

The donor DNA for SCD may be either linear or circular, but is preferably circular. Preferably, the donor DNA for SCD is a plasmid. The donor DNA for SCD may contain a spacer sequence between the above sequences.

A method for introducing the donor DNA for SCD into cells is not particularly limited, and a known method (for example, lipofection method) can be used without particular limitation.

It is preferable to simultaneously introduce the gRNA and Cas9 and the donor DNA for SCD into cells to carry out step [2-1] and step [2-2] simultaneously.

Substitution of sequences by homologous recombination can be carried out using well-known and commonly used techniques.

After homologous recombination, cells satisfying the following two conditions are selected and used in Step 3.

Condition 1: Not expressing the negative selection marker present in the allele targeted for cleavage in step [2-1] (the allele in which the positive selection marker gene targeted by the gRNA is present).

Condition 2: Expressing the negative selection marker present in the allele different from the allele targeted for cleavage in step [2-1].

In the selected cells, the target region of one allele is replaced with the SCD coding sequence, and the target region of the other allele remains replaced with the selection marker gene. In other words, in the selected cells, the alpha chain genes of HLA-F, HLA-G and HLA-A of one allele are knocked out, and instead, the SCD coding sequence is knocked in.

### [Step 3] Removal of selection marker gene from other allele

The other allele refers to the allele into which the SCD coding sequence was not introduced in Step 2.

### [3-1] Site-specific cleavage of DNA using CRISPR/Cas system in other allele

One double-strand break per allele is introduced by a CRISPR/Cas9 system using a gRNA targeting the positive selection marker gene not targeted in step [2-1] and Cas9 protein.

The sequence of the gRNA does not have to be completely identical to the target sequence as long as it can bind to the target sequence. For introduction of the gRNA, a polynucleotide encoding the gRNA (for example, an expression vector) may be used.

A method for introducing the gRNA and Cas9 into cells is not particularly limited, and a known method (for example, lipofection method) can be used without particular limitation.

Note that step [3-1] may be carried out using another site-specific nuclease, for example, TALEN, ZFN, or the like, instead of the CRISPR/Cas system.

### [3-2] Removal of selection marker gene from other allele

In order to remove the selection marker, a DNA for selection marker excision is used.

In the DNA for selection marker excision, an "upstream homology arm sequence" and a "downstream homology arm sequence" are linked, and no other sequence exists between both homology arm sequences.

The "upstream homology arm sequence" and the "downstream homology arm sequence" of the DNA for selection marker excision are the same as the "upstream homology arm sequence" and the "downstream homology arm sequence" of the donor DNA for selection marker used in step [1-2], respectively. Therefore, homologous recombination occurs between the "DNA for selection marker excision" and the "allele containing the selection marker gene," and the selection marker gene is removed from the allele. Homologous recombination can be carried out using well-known and commonly used techniques.

Note that as long as homologous recombination occurs, the "upstream homology arm sequence" and the "downstream homology arm sequence" of the DNA for selection marker excision may be different from the "upstream homology arm sequence" and the "downstream homology arm sequence" of the donor DNA for selection marker used in step [1-2], respectively.

The DNA for selection marker excision may be either linear or circular, but is preferably circular. Preferably, the DNA for selection marker excision is a plasmid.

A method for introducing the DNA for selection marker excision into cells is not particularly limited, and a known method (for example, lipofection method) can be used without particular limitation.

It is preferable to simultaneously introduce the gRNA and Cas9 and the donor DNA for SCD into cells to carry out step [3-1] and step [3-2] simultaneously.

After homologous recombination, cells expressing neither of the two types of negative selection markers introduced in step [1-2] are selected.

In the selected cells, the selection marker gene is removed from the other allele. In other words, in the selected cells, the "alpha chain genes of HLA-F, HLA-G and HLA-A" are deleted in one allele, and the "SCD coding sequence" is knocked in instead; and further, the "alpha chain genes of HLA-F, HLA-G and HLA-A" are deleted in the other allele.

In other words, for example, when the target region is the target region 2, in the selected cells, the target region 2 (chromosomal region corresponding to chr6: 29,707,019-29,956,470 of hg38 genome sequence) is deleted in both alleles.

In the cell in which the "alpha chain genes of HLA-F, HLA-G and HLA-A" are deleted from both alleles, the expression of the genes is reduced compared with a wild-type cell. The "wild-type cell" refers to a cell not subjected to the aforementioned Steps 1 to 3. Comparison of gene expression can be carried out using a known gene expression analysis technique (for example, reverse transcription quantitative PCR method).

The cell in which the "alpha chain genes of HLA-F, HLA-G and HLA-A" are deleted from both alleles may lack the expression of the genes.

The cell in which the "alpha chain genes of HLA-F, HLA-G and HLA-A" are deleted from both alleles may lack the expression of proteins encoded by the genes, or may lack the expression of proteins encoded by the genes on the cell membrane.

The reason why the SCD was introduced into only one of the alleles in Steps 1 to 3 is that it is possible to easily confirm whether the intended gene introduction has been achieved or not. Generally, confirmation of genotype is performed using PCR; however, when aiming to introduce SCD into both alleles, it is difficult to distinguish "a cell in which SCD is introduced into both alleles (target cell)" from "a cell in which SCD is introduced into only one allele (non-target cell)" by PCR.

In contrast, when aiming to introduce SCD into one allele, "a cell in which SCD is introduced into only one allele (target cell)" and "a cell in which SCD is introduced into both alleles (non-target cell)" can be easily distinguished by PCR.

However, the present invention also encompasses a gene-modified cell in which SCD is introduced into both alleles.

### 2. Method for knocking in SCT

### [Step 1] Substitution of region containing alpha chain genes of HLA-C and HLA-B with selection marker gene

The "region containing alpha chain genes of HLA-C and HLA-B" (hereinafter, also referred to as "target region") replaced in this step is, for example, a chromosomal region corresponding to chr6: 31,224,837-31,378,196 of the hg38 genome sequence (Target Region 1), or a chromosomal region corresponding to chr6: 31,227,286-31,378,780 of the hg38 genome sequence (Target Region 2).

### [1-1] Site-specific cleavage of DNA using CRISPR/Cas system

When the target region is Target Region 1, two double-strand breaks per allele are introduced by a CRISPR/Cas9 system using a guide RNA (gRNA) targeting an upstream region of the alpha chain gene of HLA-C (chr6: 31,225,752-31,225,771 of hg38 genome sequence), a gRNA targeting a downstream region of the alpha chain gene of HLA-B (chr6: 31,377,258-31,377,277 of hg38 genome sequence), and a Cas9 protein.

When the target region is Target Region 2, two double-strand breaks per allele are introduced by a CRISPR/Cas9 system using a guide RNA (gRNA) targeting an upstream region of the alpha chain gene of HLA-C (chr6: 31,228,800-31,228,822 of hg38 genome sequence), a gRNA targeting a downstream region of the alpha chain gene of HLA-B (chr6: 31,377,258-31,377,277 of hg38 genome sequence), and a Cas9 protein.

The sequence of the gRNA need not be completely identical to the target sequence as long as it can bind to the target sequence. For introduction of the gRNA, a polynucleotide encoding the gRNA (for example, an expression vector) may be used.

A method for introducing the gRNA and Cas9 into the cell is not particularly limited, and a known method (for example, a lipofection method) can be used without particular limitation.

Note that step [1-1] may be carried out using another site-specific nuclease, for example, TALEN, ZFN, or the like, instead of the CRISPR/Cas system.

### [1-2] Introduction of different selection marker genes into target region of each allele

In order to introduce the selection marker gene, a donor DNA for selection marker is used.

The "donor DNA for selection marker" contains a "selection marker gene" between an "upstream homology arm sequence" and a "downstream homology arm sequence."

The "upstream homology arm sequence" has a sequence capable of homologous recombination with an upstream sequence in the target region (for example, when the target region is Target Region 1, chr6: 31,224,837-31,225,754 of hg38 genome sequence; for example, when the target region is Target Region 2, chr6: 31,227,286-31,228,805 of hg38 genome sequence) existing in one of two chromosomes (the chromosome to be subjected to homologous recombination). The upstream homology arm sequence need not be completely identical to the sequence adjacent to the upstream side of the target region as long as homologous recombination is possible, but preferably has 90% or more, and further preferably 95% or more sequence identity.

The "downstream homology arm sequence" has a sequence capable of homologous recombination with a downstream sequence in the target region (for example, when the target region is Target Region 1, chr6: 31,377,261-31,378,196 of hg38 genome sequence; for example, when the target region is Target Region 2, chr6: 31,377,261-31,378,780 of hg38 genome sequence) existing in one of two chromosomes (the chromosome to be subjected to homologous recombination). The downstream homology arm sequence need not be completely identical to the sequence adjacent to the downstream side of the target region as long as homologous recombination is possible, but preferably has 90% or more, and further preferably 95% or more sequence identity.

Lengths of the upstream homology arm and the downstream homology arm may be, for example, about 500 bp to 3 kbp, but can be appropriately designed by a person skilled in the art in the same manner as in ordinary design of donor DNA.

The "donor DNA for selection marker" contains a positive selection marker (for example, a drug resistance gene) used in this step and a negative selection marker (for example, a fluorescent protein) used in Steps 2 and 3 described later. The selection marker can be appropriately selected according to the type of cell to be used and the like.

The selection marker may be one that serves as both a positive selection marker and a negative selection marker (dual-purpose marker). Examples of the dual-purpose marker include fluorescent proteins.

In this step, two types of donor DNAs for selection marker are used. The two types of donor DNAs for selection marker each have a mutually distinguishably different positive selection marker gene. Further, the two types of donor DNAs for selection marker each have a mutually distinguishably different negative selection marker gene. Examples of the combination of selection marker genes possessed by the two types of donor DNAs for selection marker include those described in "1. Method for knocking in SCD."

The donor DNA for selection marker may be either linear or circular, but is preferably circular. Preferably, the donor DNA for selection marker is a plasmid. The donor DNA for selection marker may contain a spacer sequence between the above sequences.

A method for introducing the donor DNA for selection marker into the cell is not particularly limited, and a known method (for example, a lipofection method) can be used without particular limitation.

It is preferable to simultaneously introduce the gRNA and Cas9 and the donor DNA for selection marker into the cell to carry out step [1-1] and step [1-2] simultaneously.

Homologous recombination (HDR) occurs between the donor DNA for selection marker having the "upstream homology arm sequence" and "downstream homology arm sequence" and the chromosome of the cell. Substitution of sequences by homologous recombination can be carried out using well-known and commonly used techniques. After homologous recombination, cells expressing two types of positive selection markers are selected and used in Step 2.

In the selected cells, the target regions of the two alleles are replaced with different types of selection marker genes, respectively. In other words, in the selected cells, the alpha chain genes of HLA-C and HLA-B of the two alleles are knocked out, and instead, different types of selection marker genes are knocked into each allele.

### [Step 2] Substitution of selection marker gene with polynucleotide encoding SCT in one allele

### [2-1] Site-specific cleavage of DNA using CRISPR/Cas system in one allele

One double-strand break per allele is introduced by a CRISPR/Cas9 system using a gRNA targeting one of the two types of positive selection marker genes used in step [1-2] and a Cas9 protein.

The sequence of the gRNA need not be completely identical to the target sequence as long as it can bind to the target sequence. For introduction of the gRNA, a polynucleotide encoding the gRNA (for example, an expression vector) may be used.

A method for introducing the gRNA and Cas9 into the cell is not particularly limited, and a known method (for example, a lipofection method) can be used without particular limitation.

Note that step [2-1] may be carried out using another site-specific nuclease, for example, TALEN, ZFN, or the like, instead of the CRISPR/Cas system.

### [2-2] Introduction of polynucleotide encoding SCT into only one allele

In order to introduce the polynucleotide encoding SCT (hereinafter, also referred to as "SCT coding sequence"), a donor DNA for SCT is used.

The "donor DNA for SCT" contains the "SCT coding sequence" (for example, SEQ ID NO: 7) between an "upstream homology arm sequence" and a "downstream homology arm sequence."

The "upstream homology arm sequence" and "downstream homology arm sequence" of the donor DNA for SCT are the same as the "upstream homology arm sequence" and "downstream homology arm sequence" of the donor DNA for selection marker used in step [1-2], respectively. Therefore, homologous recombination occurs between the "donor DNA for SCT" and the "allele containing the selection marker gene," and the selection marker gene is replaced by the SCT coding sequence. Substitution of sequences by homologous recombination can be carried out using well-known and commonly used techniques.

Note that as long as homologous recombination occurs, the "upstream homology arm sequence" and "downstream homology arm sequence" of the donor DNA for SCT may be different from the "upstream homology arm sequence" and "downstream homology arm sequence" of the donor DNA for selection marker used in step [1-2], respectively.

For the purpose of increasing the expression level of SCT, the donor DNA for SCT preferably contains a promoter sequence of an alpha chain gene of HLA-B (preferably, a sequence corresponding to chr6: 31,357,159-31,359,283 of hg38 genome sequence) upstream of the SCT coding sequence. Here, since what SCT mimics is HLA-E, use of a promoter of an alpha chain gene of HLA-E is conceivable. However, in the present invention, knockout of the alpha chain gene of HLA-E is not essential; therefore, if multiple identical promoters exist in the genome, binding of transcription factors competes, and it is considered that the expression level of SCT decreases. Therefore, it is preferable to use a promoter of an alpha chain gene of HLA-B which is known to be stably expressed in multiple cell lineages.

Further, in an embodiment in which SCD is introduced in addition to SCT, when a promoter sequence of an alpha chain gene of HLA-A is used for increasing the expression level of SCD, competition of promoters can be avoided by using the promoter sequence of the alpha chain gene of HLA-B for increasing the expression level of SCT.

In the present invention, a known promoter sequence of an alpha chain gene of HLA-B can be used. In a preferred embodiment, the promoter sequence of the alpha chain gene of HLA-B may be a sequence comprising a sequence having 80% or more, 85% or more, 90% or more, 95% or more, or 97% or more sequence identity with the following SEQ ID NO: 11.

In a particularly preferred embodiment, the promoter sequence of the alpha chain gene of HLA-B is the sequence set forth in SEQ ID NO: 11 below.

[SEQ ID NO: 11]
AGCTGTGAGAGACACATCAGAGCCCTGGGCACTGTCGCTGCCTGGAGTAGA
ACAAAAACAGGACCTGGTCAGAGCCCGCAGGAGACGTGGGACAGGAGGAA
TTATGGGGTGGGTGAGCTCCTCCACACTCCCACCCCCACCACTTACACGCAG
CCTGAGAGTAGCTCCCTCCTTTTCCACCTGTGGGAAGAAAATGTCCTGTGAG
GGCACTGGGAGGAAGCAGGGCCATGAGATCTTAGAGGAACCTCCTAGTCTT
GGACCCAAAAGGAATTTCCAGAAGTATGACTACAGACCCAGGGCAGGATCA
GGAAACACGAGGAAAGCAAGTGTGGGTCCTGGACCAACTGCCCTCCTAAGG
TCTGTCCTTAGCAGGGACCTTCCCCTGACTCATGAATGCTGGAATCAGGACC
CCAACACCACAACCATCAAGGTGATACATCCATCCTTCATTGTCACATGTGCT
GCACAAAAGAGTAAGTGCTGGCACACAGGGTCCCAGGCTGCGTTAGCCCCT

GTGTGCATGCTGCTTCCCAGTAATGAGGCAGGGAACACTTCTACCTGGGGCT
TGAAACCCCCAGTGGGACAAGAAAACCCAGACCCCACCCCTCACCCCTTCC
CTACCTGAACTCTTCCTCCTACACATCACAGCAGCGACCACAGCTCCGATGA
CCACAACTGCTAGGACAGCCAGGCCAGCAACAATGCCCACGATGGGGACGG
TGGACTGGGAAGACGGCTCTGGGAAAGGAGGGGAAGATGAGGGGCCCTGA
CCCTGCTGAAGGGCTCCTGCTTTCCCTGAGAAGAGATATGACCCCTCATCCCC
CTCCTTACCCCATCTCAGGGTGAGGGGCTTCGGCAGCCCCTCATGCTGTACAT
GGCATGTGTATCTCTGCTCTTCTCCAGAAGGCACCACCACAGCTGCCCACTTC
TGGAAGGTTCTATCTCCTGCTGGTCTGGTCTCCACAAGCTCAGTGTCCTGAGT
TTGGTCCTCGCCATCCCGCTGCCAGGTCAGTGTGATCTCCGCAGGGTAGAAA
CCCAGGGCCCAGCACCTCAGGGTGGCCTCATGGTCAGAGATGGGGTGGTGG
GTCACGTGTGTCTTTGGGGGGTCTGATGGGAAGAGTCAGAAAATTCAGGCG
CTTTGCATCTTTCATGGGACACCCTAGGACCACCCATGTGACCAGCCTGAGA
ATGGACAGGACACCTGGGGTGGGGAAGGGGCACAGAACCCAGACACCAGC
CTGGACGCAGGCACCTGGGATAATCTCCTATTCATTGGAAAGTTCGAGTCTCT
GAGCGGGGAACAGGGACTTCTGCTCCTGATCTGAGTGGAGGTAAAGTGACT
CAGAAGTGCTGGAATCAGAGCCCCAAACACACTGAGTGTGAGGCAGAGAAC
AAGGCCTGAGAGGAAAAGTCACGGTTCCCAAGGCTGCTGCAGGGGTCAAA
GGGGACCCCTGATCAGTATTCTAGGGACTGTCTTCCCCTCCATTTCCTCAGAG
ACGTCATTCCTTAATTGTCTAGAGAGAAGAGGGGGCCCTCAGAGGAAACTCA
GGAAAACTCATGCCATTCTCCATTCAACGGAGGGCGACATTCTAGCGCTGAT
CCCATTTTCCTCCTCTTCTCGTGGGAGGCCATCCCCGGCGACCTATAGGAGAT
GGGGAAGGCTCCCCACTGCCCCTGGTACCAGCGCGCTCCAGCTTGTCCTTCC
CGTTCTCCAGGTATCTGCGGAGCCACTCCACGCACTCGCCCTCCAGGTAGGC
TCTCCGCTGCTCCGCCTCACGGGCCGCCTCCCACTTGCGCTGGGTGATCTGA
GCCGCCGTGTCCGCGGCGGTCCAGGAGCGCAGGTCCTCGTTCAGGGCGATG
TAATCCTTGCCGTCGTAGGCGTACTGGTCATGCCCGCGGAGGAGGCGCCCGT
CCGGCCCCACGTCGCAGCCGTACATGCTCTGGAGGGTGTGAGACCCTGGCCC
CGGCCCCGCGGTCAGCCCAGTCCCCCGAGCCCCGCCCCGCCCCGACCAACC
CGCGGGGATTTTGGCCTCAACTGAAAATGAAACCGGGTAAACGCGCCTGGG

GCTCTCGCCGGTCGAGGGTCTGGGCGGGTCCCGCGGCCTCAGGGAGGCGGA
TCTCGGACCCGGAGACTCGGGGCGACCCGGGCCGTACGTGGGGGATGGGGA
GTCGTGACCTGCGCCCCGGGCCGGGGTCACTCACCGGCCTCGCTCTGGTTGT
AGTAGCCGCGCAGGTTCCGCAGGCTCTCTCGGTCAGTCTGTGCCTGGGCCTT
GTAGATCTGTGTGTTCCGGTCCCAATACTCCGGCCCCTCCTGCTCTATCCACG
GCGCCCGCGGCTCCTCTCTCGGACTCGCGGCGTCGCTGTCGAACCTCACGAA
CTGGGTGTCGTCCACGTAGCCCACTGAGATGAAGCGGGGCTCCCCGCGGCC
GGGCCGGGACACGGAGGTGTAGAAATACCTCATGGAGTGGGAGCC

The promoter sequence of the alpha chain gene of HLA-B is knocked in in a manner operably linked to the SCT coding sequence. "Operably linked" means that the promoter sequence of the alpha chain gene of HLA-B is disposed relative to the SCT coding sequence in a manner that controls expression of the SCT coding sequence.

In another aspect of the present invention, a promoter sequence of a gene encoding an alpha chain of an HLA Class I molecule other than HLA-E may be knocked in in a manner operably linked to the SCT coding sequence.

The donor DNA for SCT may be either linear or circular, but is preferably circular. Preferably, the donor DNA for SCT is a plasmid. The donor DNA for SCT may contain a spacer sequence between the above sequences.

A method for introducing the donor DNA for SCT into the cell is not particularly limited, and a known method (for example, a lipofection method) can be used without particular limitation.

It is preferable to simultaneously introduce the gRNA and Cas9 and the donor DNA for SCT into the cell to carry out step [2-1] and step [2-2] simultaneously.

Substitution of sequences by homologous recombination can be carried out using well-known and commonly used techniques.

After homologous recombination, cells satisfying the following two conditions are selected and used in Step 3.

Condition 1: Not expressing the negative selection marker present in the allele targeted for cleavage in step [2-1] (the allele in which the positive selection marker gene targeted by the gRNA is present).

Condition 2: Expressing the negative selection marker present in the allele distinct from the allele targeted for cleavage in step [2-1].

In the selected cells, the target region of one allele is replaced with the SCT coding sequence, and the target region of the other allele remains replaced with the selection marker gene. In other words, in the selected cells, the alpha chain genes of HLA-C and HLA-B of one allele are knocked out, and instead, the SCT coding sequence is knocked in.

### [Step 3] Removal of selection marker gene from the other allele

The other allele refers to the allele into which the SCT coding sequence was not introduced in Step 2.

### [3-1] Site-specific cleavage of DNA using CRISPR/Cas system in other allele

One double-strand break per allele is introduced by a CRISPR/Cas9 system using a gRNA targeting the positive selection marker gene not targeted in step [2-1] and a Cas9 protein.

The sequence of the gRNA need not be completely identical to the target sequence as long as it can bind to the target sequence. For introduction of the gRNA, a polynucleotide encoding the gRNA (for example, an expression vector) may be used.

The method for introducing the gRNA and Cas9 into the cell is not particularly limited, and a known method (for example, a lipofection) can be used without particular limitation.

Note that step [3-1] can be carried out using another site-specific nuclease, for example, TALEN, ZFN, or the like, instead of the CRISPR/Cas system.

### [3-2] Removal of the selection marker gene from the other allele

In order to remove the selection marker gene, DNA for the selection marker excision is used.

In the DNA for selection marker deletion, an "upstream homology arm sequence" and a "downstream homology arm sequence" are linked, and no other sequence exists between both homology arm sequences.

The "upstream homology arm sequence" and "downstream homology arm sequence" of the DNA for selection marker excision are the same as the "upstream homology arm sequence" and "downstream homology arm sequence" of the donor DNA for selection marker used in step [1-2], respectively. Therefore, homologous recombination occurs between the "DNA for selection marker excision" and the "allele containing the selection marker gene," and the selection marker gene is removed from the allele. Homologous recombination can be carried out using well-known and commonly used techniques.

Note that as long as homologous recombination occurs, the "upstream homology arm sequence" and "downstream homology arm sequence" of the DNA for selection marker excision can be different from the "upstream homology arm sequence" and "downstream homology arm sequence" of the donor DNA for selection marker used in step [1-2], respectively.

The DNA for selection marker excision may be either linear or circular, but is preferably circular. Preferably, the DNA for selection marker excision is a plasmid.

A method for introducing the DNA for selection marker excision into the cell is not particularly limited, and a known method (for example, a lipofection method) can be used without particular limitation.

It is preferable to simultaneously introduce the gRNA and Cas9 and the donor DNA for SCD into the cell to carry out step [3-1] and step [3-2] simultaneously.

After homologous recombination, cells expressing neither of the two types of negative selection markers introduced in step [1-2] are selected.

In the selected cells, the selection marker gene is removed from the other allele. In other words, in the selected cells, the "alpha chain genes of HLA-C and HLA-B" are deleted in one allele, and the "SCT coding sequence" is knocked in instead; and further, the "alpha chain genes of HLA-C and HLA-B" are deleted in the other allele.

In still other words, for example, when the target region is Target Region 2, in the selected cells, the chromosomal region corresponding to chr6: 31,228,806-31,377,277 of the hg38 genome sequence (region excluding the upstream homology arm and the downstream homology arm from Target Region 2) is deleted in both alleles.

In cells in which the "alpha chain genes of HLA-C and HLA-B" are deleted from both alleles, expression of the genes is reduced compared with wild-type cells. "Wild-type cell" refers to a cell not subjected to the aforementioned Steps 1 to 3. Comparison of gene expression can be carried out using a known gene expression analysis technique (for example, reverse transcription quantitative PCR method).

The cells in which the "alpha chain genes of HLA-C and HLA-B" are deleted from both alleles may lack the expression of the genes.

The cells in which the "alpha chain genes of HLA-C and HLA-B" are deleted from both alleles may lack the expression of proteins encoded by the genes, and may lack the expression of proteins encoded by the genes on the cell membrane.

The reason why SCT was introduced into only one allele in Steps 1 to 3 is that it is possible to easily confirm whether the intended gene introduction has been achieved or not. Generally, confirmation of genotype is performed using PCR; however, when aiming to introduce SCT into both alleles, it is difficult to distinguish "cells in which SCT is introduced into both alleles (target cells)" from "cells in which SCT is introduced into only one allele (non-target cells)" by PCR.

In contrast, when aiming to introduce SCT into one allele, "cells in which SCD is introduced into only one allele (target cells)" and "cells in which SCT is introduced into both alleles (non-target cells)" can be easily distinguished by PCR.

However, the present invention also encompasses gene-modified cells in which SCT is introduced into both alleles.

### 3. Method for knocking in SCD and SCT

SCD and SCT can be knocked in by carrying out "2. Method for knocking in SCT" subsequent to the aforementioned "1. Method for knocking in SCD," or by carrying out "1. Method for knocking in SCD" subsequent to "2. Method for knocking in SCT."

Up to this point, embodiments have been described in which the "polynucleotide encoding SCD" is knocked into the "region containing alpha chain genes of HLA-F, HLA-G and HLA-A" of the subject cell, and the "polynucleotide encoding SCT" is knocked into the "region containing alpha chain genes of HLA-C and HLA-B"; however, in another embodiment of the present invention, the "polynucleotide encoding SCT" may be knocked into the "region containing alpha chain genes of HLA-F, HLA-G and HLA-A" of the subject cell, and the "polynucleotide encoding SCD" may be knocked into the "region containing alpha chain genes of HLA-C and HLA-B."

Accordingly, the promoter used in a manner operably linked to SCD or SCT in each aspect of the present invention described above can be similarly used for knock-in in a manner operably linked to any sequence including the SCD coding sequence or the SCT coding sequence, respectively. For example, in another aspect of the present invention, the promoter sequence of the alpha chain gene of HLA-A may be knocked in in a manner operably linked to the SCT coding sequence. Further, in another aspect of the present invention, the promoter sequence of the alpha chain gene of HLA-B may be knocked in in a manner operably linked to the SCD coding sequence.

In the above, the procedure of simultaneously carrying out (i) deletion (deficiency) of the alpha chain genes of HLA Class I molecules and (ii) introduction (knock-in) of SCD and/or SCT has been described; however, the following different procedure may be adopted.

A cleavage is introduced into a region adjacent to an alpha chain gene cluster region of HLA Class I molecules (a series of regions containing alpha chain genes of HLA-F, HLA-G and HLA-A, or a series of regions containing alpha chain genes of HLA-C and HLA-B, etc.) (hereinafter, also referred to as "adjacent region"), and a polynucleotide encoding SCD and/or SCT and a selection marker gene (selected from a positive selection marker gene and a negative selection marker gene, or a marker gene serving as both positive selection and negative selection) can be introduced into the adjacent region such that the selection marker gene is disposed closer to the alpha chain gene cluster region of HLA Class I molecules than one or both of the polynucleotides encoding SCD and/or SCT.

As described above, by mounting the polynucleotide encoding SCD and/or SCT and the selection marker gene on one of the donor DNAs for selection marker, and mounting a marker gene mutually distinguishably different from the selection marker gene on the other donor DNA for selection marker, and obtaining cells expressing respective selection marker genes, the polynucleotide encoding SCD and/or SCT and the selection marker gene can be introduced into one allele of the subject cell, and the selection marker gene can be introduced into the other allele.

Thereafter, by deleting a contiguous region (series of regions) including the selection marker gene and the alpha chain gene cluster region of HLA Class I molecules from the one allele, the region containing the alpha chain genes of HLA Class I molecules can be deleted. Preferably, by additionally deleting a region containing the selection marker gene from the other allele, it is possible to leave no region containing the alpha chain genes of HLA Class I molecules on the genome of the subject cell.

Deletion of the contiguous region and the region containing the selection marker gene can be achieved by sequence-specifically cleaving two sites sandwiching the respective regions. In this deletion, it is not necessary to use a donor DNA. Cells in which the above contiguous region has been deleted can be obtained as cells not expressing the selection marker gene (selected from a positive selection marker gene and a negative selection marker gene, or a marker gene serving as both positive selection and negative selection).

By analyzing genes of the obtained cells, it is possible to confirm that SCD and/or SCT has been introduced and the contiguous region has been deleted.

In addition, gene editing can be carried out in various different orders.

### [Modification for reducing expression of CIITA gene]

CIITA (MHC class II transactivator) is a transcription factor that regulates expression of HLA Class II molecules.

HLA Class II molecules are expressed exclusively in immune cells such as monocytes, macrophages, dendritic cells, and B cells, and present non-self-derived peptides existing extracellularly to helper T cells to activate immunity.

When recognizing the presented antigen as non-self, helper T cells cause an immune reaction by releasing interferon that activates killer T cells, thereby damaging transplanted cells (Transplant Direct. 2017 May; 3(5): e152).

Therefore, in a preferred embodiment of the present invention, the CIITA gene is deleted (knocked out) to suppress expression of HLA Class II molecules.

Hereinafter, a method for knocking out the CIITA gene of a diploid cell having two alleles will be described.

### 4. Method for knocking out CIITA gene

### [Step 1] Substitution of region containing CIITA gene with selection marker gene

The "region containing CIITA gene" (hereinafter, also referred to as "target region") replaced in this step includes a "region corresponding to the CIITA gene region on chr16 of the hg38 genome sequence" shown below.

| | Position on chr16 of hg38 genome sequence |
|---|---|
| CIITA | 10,877,202-10,936,394 |

The "region containing CIITA gene" may contain an upstream region of the CIITA gene (region corresponding to a region upstream of 10,877,202 on chr16 of hg38 genome sequence) and/or a downstream region of the CIITA gene (region corresponding to a region downstream of 10,936,394 on chr16 of hg38 genome sequence).

Length of the upstream region of the CIITA gene is not particularly limited as long as the method for knocking out the CIITA gene described later can be carried out, but is, for example, 800 to 20,000 nucleotides, preferably 1,000 to 5,000 nucleotides. The length of the upstream region can be appropriately set in consideration of the design position of the guide RNA, the fact that the homology arm is a specific sequence, and the like.

Length of the downstream region of the CIITA gene is not particularly limited as long as the method for knocking out the CIITA gene described later can be carried out, but is, for example, 800 to 20,000 nucleotides, preferably 1,000 to 5,000 nucleotides. The length of the downstream region can be appropriately set in consideration of the design position of the guide RNA, the fact that the homology arm is a specific sequence, and the like.

In a preferred embodiment of the present invention, the "region containing CIITA gene" is a chromosomal region corresponding to chr16: 10,872,423-10,926,506 of the hg38 genome sequence (Target Region 1), or a chromosomal region corresponding to chr16: 10,873,026-10,925,966 of the hg38 genome sequence (Target Region 2).

### [1-1] Site-specific cleavage of DNA using CRISPR/Cas system

Two double-strand breaks per allele are introduced by a CRISPR/Cas9 system using a guide RNA (gRNA) targeting an upstream region of the CIITA gene (when the target region is Target Regions 1 and 2, chr16: 10,873,824-10,873,846 of hg38 genome sequence), a gRNA targeting a downstream region of the CIITA gene (when the target region is Target Regions 1 and 2, chr16: 10,925,149-10,925,171 of hg38 genome sequence), and a Cas9 protein.

The sequence of the gRNA need not be completely identical to the target sequence as long as it can bind to the target sequence. For introduction of the gRNA, a polynucleotide encoding the gRNA (for example, an expression vector) may be used.

A method for introducing the gRNA and Cas9 into the cell is not particularly limited, and a known method (for example, a lipofection method) can be used without particular limitation.

Note that step [1-1] may be carried out using another site-specific nuclease, for example, TALEN, ZFN, or the like, instead of the CRISPR/Cas system.

### [1-2] Introduction of different selection marker genes into target region of each allele

In order to introduce the selection marker gene, a donor DNA for selection marker is used.

The "donor DNA for selection marker" contains a "selection marker gene" between an "upstream homology arm sequence" and a "downstream homology arm sequence."

The "upstream homology arm sequence" has a sequence capable of homologous recombination with an upstream sequence in the target region (for example, when the target region is Target Region 1, chr16: 10,872,423-10,873,829 of hg38 genome sequence; for example, when the target region is Target Region 2, chr16: 10,873,026-10,873,829 of hg38 genome sequence) existing in one of two chromosomes (the chromosome to be subjected to homologous recombination). The upstream homology arm sequence need not be completely identical to the sequence adjacent to the upstream side of the target region as long as homologous recombination is possible, but preferably has 90% or more, and further preferably 95% or more sequence identity.

The "downstream homology arm sequence" has a sequence capable of homologous recombination with a downstream sequence in the target region (for example, when the target region is Target Region 1, chr16: 10,925,166-10,926,506 of hg38 genome sequence; for example, when the target region is Target Region 2, chr16: 10,925,155-10,925,966 of hg38 genome sequence) existing in one of two chromosomes (the chromosome to be subjected to homologous recombination). The downstream homology arm sequence need not be completely identical to the sequence adjacent to the downstream side of the target region as long as homologous recombination is possible, but preferably has 90% or more, and further preferably 95% or more sequence identity.

Lengths of the upstream homology arm and the downstream homology arm may be, for example, about 500 bp to 3 kbp, but can be appropriately designed by a person skilled in the art in the same manner as in ordinary design of donor DNA.

The "donor DNA for selection marker" contains a positive selection marker (for example, a drug resistance gene) used in this step and a negative selection marker (for example, a fluorescent protein) used in Steps 2 and 3 described later. The selection marker can be appropriately selected according to the type of cell to be used and the like.

The selection marker may be one that serves as both a positive selection marker and a negative selection marker (dual-purpose marker). Examples of the dual-purpose marker include fluorescent proteins.

In this step, two types of donor DNAs for selection marker are used. The two types of donor DNAs for selection marker each have a mutually distinguishably different positive selection marker gene. Further, the two types of donor DNAs for selection marker each have a mutually distinguishably different negative selection marker gene. Examples of the combination of selection marker genes possessed by the two types of donor DNAs for selection marker include those described in "1. Method for knocking in SCD."

The donor DNA for selection marker may be either linear or circular, but is preferably circular. Preferably, the donor DNA for selection marker is a plasmid. The donor DNA for selection marker may contain a spacer sequence between the above sequences.

A method for introducing the donor DNA for selection marker into the cell is not particularly limited, and a known method (for example, a lipofection method) can be used without particular limitation.

It is preferable to simultaneously introduce the gRNA and Cas9 and the donor DNA for selection marker into the cell to carry out step [1-1] and step [1-2] simultaneously.

Homologous recombination (HDR) occurs between the donor DNA for selection marker having the "upstream homology arm sequence" and "downstream homology arm sequence" and the chromosome of the cell. Substitution of sequences by homologous recombination can be carried out using well-known and commonly used techniques. After homologous recombination, cells expressing two types of positive selection markers are selected and used in Step 2.

In the selected cells, the target regions of the two alleles are replaced with different types of selection marker genes, respectively. In other words, in the selected cells, the CIITA genes of the two alleles are knocked out, and instead, different types of selection marker genes are knocked into each allele.

### [Step 2] Deletion of selection marker gene from both alleles

### [2-1] Site-specific cleavage of DNA using CRISPR/Cas system

One double-strand break per allele is introduced by a CRISPR/Cas9 system using two types of gRNAs targeting each of the two types of positive selection marker genes used in step [1-2], and a Cas9 protein.

The sequence of the gRNA need not be completely identical to the target sequence as long as it can bind to the target sequence. For introduction of the gRNA, a polynucleotide encoding the gRNA (for example, an expression vector) may be used.

A method for introducing the gRNA and Cas9 into the cell is not particularly limited, and a known method (for example, a lipofection method) can be used without particular limitation.

Note that step [2-1] may be carried out using another site-specific nuclease, for example, TALEN, ZFN, or the like, instead of the CRISPR/Cas system.

### [2-2] Deletion of selection marker gene from both alleles

In order to remove the selection marker, a DNA for selection marker excision is used.

In the DNA for selection marker excision, an "upstream homology arm sequence" and a "downstream homology arm sequence" are linked, and no other sequence exists between both homology arm sequences.

The "upstream homology arm sequence" and "downstream homology arm sequence" of the DNA for selection marker excision are the same as the "upstream homology arm sequence" and "downstream homology arm sequence" of the donor DNA for selection marker used in step [1-2], respectively. Therefore, homologous recombination occurs between the "DNA for selection marker excision" and the "allele containing the selection marker gene," and the selection marker gene is removed from the allele. Homologous recombination can be carried out using well-known and commonly used techniques.

Note that as long as homologous recombination occurs, the "upstream homology arm sequence" and "downstream homology arm sequence" of the DNA for selection marker excision may be different from the "upstream homology arm sequence" and "downstream homology arm sequence" of the donor DNA for selection marker used in step [1-2], respectively.

The DNA for selection marker excision may be either linear or circular, but is preferably circular. Preferably, the DNA for selection marker excision is a plasmid.

A method for introducing the DNA for selection marker excision into the cell is not particularly limited, and a known method (for example, a lipofection method) can be used without particular limitation.

It is preferable to simultaneously introduce the gRNA and Cas9 and the donor DNA for SCD into the cell to carry out step [2-1] and step [2-2] simultaneously.

After homologous recombination, cells expressing neither of the two types of negative selection markers introduced in step [1-2] are selected.

In the selected cells, the selection marker gene is removed from both alleles. In other words, in the selected cells, the CIITA gene is knocked out in both alleles.

In still other words, for example, when the target region is Target Region 2, in the selected cells, the chromosomal region corresponding to chr16: 10,873,830-10,925,154 of the hg38 genome sequence (region excluding the upstream homology arm and the downstream homology arm from Target Region 2) is deleted in both alleles.

In cells in which the "CIITA gene" is deleted from both alleles, expression of the gene is reduced compared with wild-type cells. "Wild-type cell" refers to a cell not subjected to the aforementioned Steps 1 to 2. Comparison of gene expression can be carried out using a known gene expression analysis technique (for example, reverse transcription quantitative PCR method).

The cells in which the "CIITA gene" is deleted from both alleles may lack the expression of the gene.

The cells in which the "CIITA gene" is deleted from both alleles may lack the expression of proteins encoded by the gene, and may lack the expression of proteins encoded by the gene on the cell membrane.

In one aspect of the present invention, expression of HLA Class II molecules may be reduced by means other than deletion (knockout) of the aforementioned CIITA gene, for example, by deleting an endogenous gene encoding RFXANK, which is one of transcription control factors of HLA Class II molecule genes, or by deleting an endogenous gene of an HLA Class II molecule.

In the cells of the present invention, expression of an alpha chain of the aforementioned predetermined HLA Class I molecule (and consequently expression of a functional HLA Class I molecule having antigen-presenting ability) is suppressed; therefore, rejection reaction due to attacks from T cells (particularly, CD8⁺ T cells and/or CD4⁺ T cells) of a transplant recipient is alleviated compared with wild-type cells. "Wild-type cell" refers to a cell not subjected to the aforementioned step of knocking in SCD and/or SCT. Comparison of rejection reaction can be evaluated using, for example, proliferation of CD8⁺ T cells and/or CD4⁺ T cells as an index.

Further, since the cells of the present invention express a single-chain fusion peptide (SCD and/or SCT) mimicking HLA-G or HLA-E which functions in an inhibitory manner against the activity (cytotoxicity) of NK cells or macrophages, rejection reaction due to attacks from NK cells and/or macrophages of a transplant recipient is alleviated compared with wild-type cells. "Wild-type cell" refers to a cell not subjected to the aforementioned step of knocking in SCD and/or SCT. Comparison of rejection reaction can be evaluated using, for example, cytotoxicity by NK cells and/or macrophages as an index.

### [Other HLA related factors]

In one aspect of the present invention, the cell of the present invention may be a cell retaining a B2M gene. Here, the cell retaining a B2M gene may be a cell retaining a wild-type B2M gene, or may be a gene-modified cell retaining a B2M gene possessed by the cell before gene modification. Further, the cell retaining a B2M gene may be a cell in which a protein encoded by the B2M gene is expressed.

### [Use of gene-modified cells]

In the cells of the present invention subjected to the aforementioned gene modification, attacks from T cells and the like (particularly, CD8⁺ T cells and CD4⁺ T cells) are avoided by suppression of expression of predetermined HLA Class I molecules, and attacks from NK cells and the like are avoided by expression of SCD and/or SCT. Due to such characteristics, cells having improved histocompatibility with a transplant recipient can be used as cells for transplantation for regenerative medicine (particularly allogeneic transplantation).

The cells of the present invention obtained by subjecting pluripotent stem cells such as iPS cells to the aforementioned gene modification can be transplanted after being differentiated according to the purpose of transplantation or the like. Examples of differentiated cells include immune cells, cardiomyocytes, nerve cells, and the like. Therefore, a cell derived from the HLA gene-modified cell subjected to gene modification according to the present invention (for example, a differentiated cell) is also one aspect of the present invention.

### [Knock-in of effector cell inhibitory factor]

Up to this point, embodiments using SCD and/or SCT, which are factors that avoid recognition by NK cells and macrophages and suppress attacks by them (effector cell inhibitory factors), as factors to be knocked in have been described; however, in another aspect of the present invention, other effector cell inhibitory factors can be used for knock-in in addition to SCD and/or SCT. Further, in another aspect of the present invention, other effector cell inhibitory factors can be used for knock-in instead of SCD and SCT.

That is, in these aspects, one or more kinds of effector cell inhibitory factors including SCD and SCT are knocked in. This aspect includes the following HLA gene-modified cells 1 to 3.
1. An HLA gene-modified cell, wherein
   a polynucleotide encoding an effector cell inhibitory factor is knocked into a region containing alpha chain genes of HLA-F, HLA-G and HLA-A, and
   further, alpha chain genes of HLA-F, HLA-G and HLA-A of the cell before gene modification are deleted.
2. An HLA gene-modified cell, wherein
   a polynucleotide encoding an effector cell inhibitory factor is knocked into a region containing alpha chain genes of HLA-C and HLA-B, and
   further, alpha chain genes of HLA-C and HLA-B of the cell before gene modification are deleted.
3. An HLA gene-modified cell, wherein
   the following polynucleotide (1) is knocked into a region containing alpha chain genes of HLA-F, HLA-G and HLA-A,
      (1) a polynucleotide encoding a first effector cell inhibitory factor;
         the following polynucleotide (2) is knocked into a region containing alpha chain genes of HLA-C and HLA-B,
      (2) a polynucleotide encoding a second effector cell inhibitory factor which is identical to or different from the first effector cell inhibitory factor; and
   further, alpha chain genes of HLA-A, HLA-B, HLA-C, HLA-F and HLA-G of the cell before gene modification are deleted.

Examples of the "effector cell" include NK cells, macrophages, and the like.

Examples of the "effector cell inhibitory factor" include known factors capable of avoiding recognition by effector cells and suppressing cytotoxicity thereof. Specific examples include NK cell inhibitory factors (for example, HLA-E, HLA-G, PD-L1, CD47, CD68, and ICAM-1) and macrophage inhibitory factors (for example, HLA-E and HLA-G).

The "effector cell inhibitory factor" may be a variant of a naturally occurring factor.

Examples of the variant include fusion peptides comprising an alpha chain of HLA-G and beta-2 microglobulin (for example, single-chain dimer fusion peptides, more specifically SCD).

Other examples of the variant include fusion peptides comprising an alpha chain of HLA-E and beta-2 microglobulin, and fusion peptides comprising an alpha chain of HLA-E, beta-2 microglobulin, and a signal peptide of HLA (specifically, a signal peptide of an HLA Class I molecule, for example, a signal peptide of HLA-A, HLA-B, HLA-C or HLA-G) (for example, single-chain trimer fusion peptides, more specifically SCT).

Knock-in of the polynucleotide encoding the effector cell inhibitory factor can be carried out using a homologous recombination method using a site-specific nuclease (for example, methods described in eLife, 2017, 6, e27873, FEBS Letters, 591, 2017, 903-913 and International Publication No. WO 20211206054), similarly to the knock-in of the polynucleotide encoding SCD and/or SCT described above.

The region into which the polynucleotide encoding the effector cell inhibitory factor is knocked in is the same as the "region containing alpha chain genes of HLA-F, HLA-G and HLA-A" and/or the "region containing alpha chain genes of HLA-C and HLA-B" described regarding the knock-in of SCD and/or SCT.

In another aspect, a polynucleotide encoding one or more kinds of effector cell inhibitory factors is knocked into only one allele of the subject cell.

In another aspect, a polynucleotide encoding one or more kinds of effector cell inhibitory factors is knocked into each allele of the subject cell, respectively.

In another aspect, one or more kinds of promoter sequences of alpha chain genes of HLA (for example, HLA-F, HLA-G, HLA-A, HLA-B or HLA-C) are knocked in together with the polynucleotide encoding the effector cell inhibitory factor.

In another aspect, one or more kinds of promoter sequences whose expression is induced by IFN gamma are knocked in together with the polynucleotide encoding the effector cell inhibitory factor.

The promoter sequence is knocked in in a manner operably linked to the polynucleotide encoding the effector cell inhibitory factor. "Operably linked" means that the promoter sequence is disposed relative to the polynucleotide in a manner that controls expression of the polynucleotide encoding the effector cell inhibitory factor.

### [Method for producing HLA gene-modified cell]

The present invention also relates to a method for producing the aforementioned HLA gene-modified cell.

The production method of the present invention comprises a step of introducing (knocking in) a polynucleotide encoding an effector cell inhibitory factor (for example, SCD and SCT) into a region encoding an alpha chain of an HLA Class I molecule (target region) of a subject cell using a gene modification technique. As the gene modification technique, gene modification utilizing a site-specific nuclease (for example, the homologous recombination method described above in the present specification) can be used.

One aspect of the production method of the present invention comprises a step of knocking in a polynucleotide encoding an effector cell inhibitory factor into only one allele of the subject cell. This aspect may further comprise a step of selecting a cell in which a target sequence has been introduced into only one allele by introducing two types of selection markers into target regions of two alleles of the subject cell at the time of knock-in.

One aspect of the production method of the present invention (hereinafter, also referred to as "one aspect of the present invention") comprises a step of knocking in a polynucleotide encoding an effector cell inhibitory factor into each allele of the subject cell, respectively.

One aspect of the present invention comprises a step of knocking in one or more kinds of fusion peptides selected from a fusion peptide comprising beta-2 microglobulin and an alpha chain of HLA-G and a fusion peptide comprising beta-2 microglobulin and an alpha chain of HLA-E into one or more kinds of regions selected from a region containing alpha chain genes of HLA-F, HLA-G and HLA-A and a region containing alpha chain genes of HLA-C and HLA-B. Preferably, the fusion peptide comprising beta-2 microglobulin and an alpha chain of HLA-G is a single-chain dimer (SCD) fusion peptide comprising beta-2 microglobulin and an alpha chain of HLA-G. Further, preferably, the fusion peptide comprising beta-2 microglobulin and an alpha chain of HLA-E is a single-chain trimer fusion peptide comprising a signal peptide of HLA Class I, beta-2 microglobulin, and an alpha chain of HLA-E, and more preferably a single-chain trimer (SCT) fusion peptide comprising a signal peptide of HLA-A, HLA-B, HLA-C or HLA-G, beta-2 microglobulin, and an alpha chain of HLA-E.

One aspect of the present invention comprises a step of knocking in a polynucleotide encoding a single-chain dimer (SCD) fusion peptide comprising beta-2 microglobulin and an alpha chain of HLA-G into a region containing alpha chain genes of HLA-F, HLA-G and HLA-A of the subject cell.

One aspect of the present invention comprises a step of knocking in a polynucleotide encoding a single-chain trimer fusion peptide comprising a signal peptide of HLA Class I, beta-2 microglobulin, and an alpha chain of HLA-E into a region containing alpha chain genes of HLA-C and HLA-B of the subject cell, and more preferably comprises a step of knocking in a polynucleotide encoding a single-chain trimer (SCT) fusion peptide comprising a signal peptide of HLA-A, HLA-B, HLA-C or HLA-G, beta-2 microglobulin, and an alpha chain of HLA-E into a region containing alpha chain genes of HLA-C and HLA-B of the subject cell.

One aspect of the present invention comprises a step of knocking in the polynucleotide encoding the SCD and the polynucleotide encoding the SCT into regions containing alpha chain genes of HLA-A, HLA-B, HLA-C, HLA-F and HLA-G of the subject cell.

In one aspect of the present invention, the region containing alpha chain genes of HLA-F, HLA-G and HLA-A is a chromosomal region corresponding to a chromosomal region of chr6: 29,704,906-29,958,642 of the hg38 genome sequence.

In one aspect of the present invention, the region containing alpha chain genes of HLA-C and HLA-B is a chromosomal region corresponding to a chromosomal region of chr6: 31,224,837-31,378,196 of the hg38 genome sequence.

In one aspect of the present invention, the region containing alpha chain genes of HLA-C and HLA-B is a chromosomal region corresponding to a chromosomal region of chr6: 31,227,286-31,378,780 of the hg38 genome sequence.

One aspect of the present invention further comprises a step of knocking out an HLA Class II molecule gene of the subject cell.

In one aspect of the present invention, the HLA Class II molecule gene is a CIITA gene.

In one aspect of the present invention, the subject cell is a human pluripotent stem cell.

In one aspect of the present invention, the subject cell is a human iPS cell or ES cell.

In one aspect of the present invention, the subject cell is a human iPS cell line 253G4.

In one aspect of the present invention, the subject cell is a human iPS cell line 201B7.

In one aspect of the present invention, the subject cell is a cell obtained by differentiating a human pluripotent stem cell. In one aspect of the present invention, the human pluripotent stem cell is a human iPS cell or ES cell. In one aspect of the present invention, the human pluripotent stem cell is a human iPS cell line 253G4 or a human iPS cell or ES cell. In one aspect of the present invention, the cell obtained by differentiating a human pluripotent stem cell is a T cell or an NK cell.

In one aspect of the present invention, the polynucleotide encoding SCD comprises a nucleotide sequence set forth in SEQ ID NO: 2 or 5.

In one aspect of the present invention, the polynucleotide encoding SCT comprises a nucleotide sequence set forth in SEQ ID NO: 7.

In one aspect of the present invention, the step of knocking in the polynucleotide encoding SCD comprises a step of knocking in a promoter sequence of an alpha chain gene of HLA-A together with the polynucleotide encoding SCD, and the promoter sequence is operably linked to a sequence encoding SCD. In one aspect of the present invention, the promoter sequence of the alpha chain gene of HLA-A comprises a nucleotide sequence set forth in SEQ ID NO: 10.

In one aspect of the present invention, the step of knocking in the polynucleotide encoding SCT comprises a step of knocking in a promoter sequence of an alpha chain gene of HLA-B together with the polynucleotide encoding SCT, and the promoter sequence is operably linked to a sequence encoding SCT. In one aspect of the present invention, the promoter sequence of the alpha chain gene of HLA-B comprises a nucleotide sequence set forth in SEQ ID NO: 11.

In one aspect of the present invention, the production method does not comprise a step of making a B2M gene deficient.

In one aspect of the present invention, the production method does not comprise a step of deleting a B2M gene.

In one aspect of the present invention, the production method does not comprise a step of reducing or eliminating expression of a B2M gene. The step of reducing or eliminating expression of a B2M gene includes, for example, a step of introducing a mutation that reduces or deletes expression of a B2M gene.

In one aspect of the present invention, the production method does not comprise a step of reducing or eliminating expression of a protein encoded by a B2M gene.

In one aspect of the present invention, the production method does not comprise a step of reducing or eliminating a function of a protein encoded by a B2M gene. The step of reducing or eliminating a function of a protein encoded by a B2M gene includes, for example, a step of introducing a mutation that reduces or deletes a function of a protein encoded by a B2M gene.

Aspects arbitrarily combining two or more of the aspects described above are also included in the production method of the present invention.

Further, in addition to the aspects described above, aspects provided with one or any combination of two or more of the features in production method described in the section of [Knock-in of polynucleotide encoding SCD and/or SCT] of the present specification are also included in the production method of the present invention.

### Examples

Hereinafter, the present invention will be described in further detail by way of examples, but the present invention is not limited thereto.

### [Example 1: Production of HLA gene-modified cell in which region containing alpha chain genes of HLA-F, HLA-G and HLA-A was replaced (knocked in) by SCD coding sequence]

HLA gene-modified cells were produced according to the method described in the aforementioned "1. Method for knocking in SCD." The "region containing alpha chain genes of HLA-F, HLA-G and HLA-A" was a chromosomal region corresponding to the chromosomal region of chr6: 29,704,906-29,958,642 of the hg38 genome sequence (Target Region 1). In addition, a CRISPR/Cas system was used as a site-specific nuclease.

As cells to be subjected to gene modification, human iPS cell line 253G4 (HLA homozygous donor-derived iPS cell stock of Kyoto University iPS Cell Research Foundation) (diploid) was used.

### Step [1-1]

A CRISPR/Cas9 system using a guide RNA (gRNA) targeting an upstream region of the alpha chain gene of HLA-F (chr6: 29,707,002-29,707,024 of hg38 genome sequence), a gRNA targeting a downstream region of the alpha chain gene of HLA-A (chr6: 29,956,465-29,956,487 of hg38 genome sequence), and a Cas9 protein was used.

The sequences of the gRNAs were completely identical to the target sequences.

An expression vector was used for introduction of the gRNA.

A lipofection method was used for introduction of the gRNA and Cas9 into the cells.

### Step [1-2]

The upstream homology arm sequence was designed targeting an upstream sequence in the target region (chr6: 29,704,906-29,707,018 of hg38 genome sequence) present in one of two chromosomes (the chromosome to be subjected to homologous recombination).

The downstream homology arm sequence was designed targeting a downstream sequence in the target region (chr6: 29,956,471-29,958,642 of hg38 genome sequence) present in one of two chromosomes (the chromosome to be subjected to homologous recombination).

The following combinations of selection marker genes possessed by two types of donor DNAs for selection marker were used.

| | Positive selection marker | Negative selection marker |
|---|---|---|
| One donor DNA for selection marker | Puromycin resistance gene | Green fluorescent protein |
| Other donor DNA for selection marker | Blasticidin resistance gene | Red fluorescent protein |

The donor DNAs for selection marker were plasmids.

A lipofection method was used for introduction of the donor DNAs for selection marker into the cells.

In this example, step [1-1] and step [1-2] were carried out simultaneously.

### [Step 2-1]

Among the two types of positive selection marker genes, a gRNA targeting the blasticidin resistance gene was used.

The sequence of the gRNA was completely identical to the target sequence. An expression vector encoding the gRNA was used for introduction of the gRNA. A lipofection method was used for introduction of the gRNA and Cas9 into the cells.

### [Step 2-2]

As the SCD coding sequence, the nucleotide sequence described in SEQ ID NO: 5 was used.

The donor DNA for SCD contained the promoter sequence of the alpha chain gene of HLA-A described in SEQ ID NO: 10.

The donor DNA for SCD was a plasmid.

A lipofection method was used for introduction of the donor DNA for SCD into the cells.

In this example, step [2-1] and step [2-2] were carried out simultaneously.

### [Step 3-1]

Among the two types of positive selection marker genes, a gRNA targeting the puromycin resistance gene was used.

The sequence of the gRNA was completely identical to the target sequence. An expression vector encoding the gRNA was used for introduction of the gRNA. A lipofection method was used for introduction of the gRNA and Cas9 into the cells.

### [Step 3-2]

The DNA for selection marker excision was a plasmid.

A lipofection method was used for introduction of the DNA for selection marker excision into the cells.

In this example, step [3-1] and step [3-2] were carried out simultaneously.

### [Expression analysis of SCD coding sequence in gene-modified cells]

Expression of the SCD coding sequence in the gene-modified cells produced in the aforementioned example was analyzed using the expression of the alpha chain gene of HLA-G contained in the SCD coding sequence as an index.

Using a 6-well plate, the cells before gene modification (human iPS cell line 253G4) or three strains of the gene-modified cells (AL-UDC (ver. 0.1)) were seeded at a cell density of 3 x 10⁵ cells/mL in 1.5 mL of StemFit AK02N medium (Ajinomoto Co., Inc.) containing 10 micro M Rho-associated kinase inhibitor (Y-27632), and cultured at 37°C and 5% CO₂.

On the next day and the day after next of seeding, the medium was replaced with StemFit AK02N medium (2 mL) supplemented with 50 ng/mL Interferon gamma (IFN gamma), and culture was continued. IFN gamma was used to stimulate the promoter of the alpha chain gene of HLA-A to express the SCD coding sequence.

After 3 days of culture, the supernatant was removed with an aspirator, and then washing was performed twice with 2 mL of DPBS(-) (D-PBS(-) without Ca and Mg, liquid, Nacalai). 0.5 x TrypLE (TrypLE^{™} Select Enzyme (1X), no phenol red, Gibco)/DPBS was added, and incubation was performed at 37°C and 5% CO₂ for about 3 minutes.

After the supernatant was removed with an aspirator, the cells were suspended in 1 mL of DPBS and centrifuged at 300 g for 3 to 5 minutes. The cells were suspended again in 1 mL of DPBS, dispensed in 250 micro L aliquots, and centrifuged at 300 g for 3 to 5 minutes. The supernatant was removed, and RNA was extracted using RNeasy Mini Kit (250) (QIAGEN).

Using PrimeScript RT reagent Kit with gDNA Eraser (TaKaRa), a reverse transcription reaction was carried out in a 10 micro L reaction system using 200 ng of RNA as a template. After the reaction, the reaction product was diluted with 40 micro L of ultrapure water, dispensed in 5 micro L aliquots, and 45 micro L of ultrapure water was added (diluted cDNA).

For one sample, 3.3 micro L of DEPC treated water, 7.5 micro L of TB Green (registered trademark) Premix Ex Taq(TM) II (Tli RNaseH Plus) (TaKaRa), 1.2 micro L of 2.5 micro M each primer mix (mixture of three types of primers (Primer 1, Primer 2 and Primer 3) amplifying the ORF region of the alpha chain gene of HLA-G), and 3 micro L of diluted cDNA were mixed and added to a 96-well plate. qPCR was carried out with a program of 95°C for 30 seconds, 40 cycles of 95°C for 5 seconds and 60°C for 30 seconds, and 95°C for 15 seconds, 60°C for 30 seconds, and 95°C for 15 seconds, to quantify the expression level of the alpha chain gene of HLA-G.

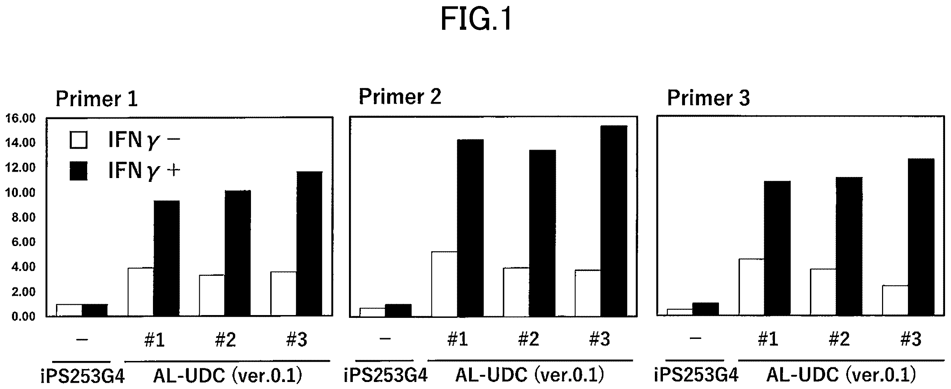

Regarding the expression of the alpha chain gene of HLA-G, the relative expression level in the gene-modified cells (AL-UDC (ver. 0.1)) when the expression level in the cells before gene modification (iPS 253G4) was taken as 1 is shown in Fig. 1.

Unlike the cells before gene modification (iPS 253G4), in the gene-modified cells (AL-UDC (ver. 0.1)), the expression level of the alpha chain gene of HLA-G increased after IFN gamma addition (IFN gamma⁺) compared with before IFN gamma addition (IFN gamma⁻). This result indicates that the SCD coding sequence was knocked in expressibly in the gene-modified cells created in the example.

### [Example 2: Production of HLA gene-modified cell in which region containing alpha chain genes of HLA-F, HLA-G and HLA-A was replaced (knocked in) by SCD coding sequence, and region containing alpha chain genes of HLA-C and HLA-B was replaced (knocked in) by SCT coding sequence]

Subsequent to the aforementioned "1. Method for knocking in SCD," "2. Method for knocking in SCT" was carried out to produce HLA gene-modified cells.

As cells to be subjected to gene modification, human iPS cell line 201B7 (HLA homozygous donor-derived iPS cell stock of Kyoto University iPS Cell Research Foundation) (diploid) was used.

### [Example 2-1: Knock-in of SCD]

The SCD coding sequence was knocked into the "region containing alpha chain genes of HLA-F, HLA-G and HLA-A" of the cells according to the method described in the aforementioned "1. Method for knocking in SCD." The "region containing alpha chain genes of HLA-F, HLA-G and HLA-A" was a chromosomal region corresponding to the chromosomal region of chr6: 29,704,906-29,958,642 of the hg38 genome sequence (Target Region 1). In addition, a CRISPR/Cas system was used as a site-specific nuclease.

### Step [1-1]

A CRISPR/Cas9 system using a guide RNA (gRNA) targeting an upstream region of the alpha chain gene of HLA-F (chr6: 29,707,002-29,707,024 of hg38 genome sequence), a gRNA targeting a downstream region of the alpha chain gene of HLA-A (chr6: 29,956,465-29,956,487 of hg38 genome sequence), and a Cas9 protein was used.

The sequences of the gRNAs were completely identical to the target sequences.

An expression vector was used for introduction of the gRNA.

A lipofection method was used for introduction of the gRNA and Cas9 into the cells.

### Step [1-2]

The upstream homology arm sequence was designed targeting an upstream sequence in the target region (chr6: 29,704,906-29,707,018 of hg38 genome sequence) present in one of two chromosomes (the chromosome to be subjected to homologous recombination).

The downstream homology arm sequence was designed targeting a downstream sequence in the target region (chr6: 29,956,471-29,958,642 of hg38 genome sequence) present in one of two chromosomes (the chromosome to be subjected to homologous recombination).

The following combinations of selection marker genes possessed by two types of donor DNAs for selection marker were used.

| | Positive selection marker | Negative selection marker |
|---|---|---|
| One donor DNA for selection marker | Puromycin resistance gene | Green fluorescent protein |
| Other donor DNA for selection marker | Blasticidin resistance gene | Red fluorescent protein |

The donor DNAs for selection marker were plasmids.

A lipofection method was used for introduction of the donor DNAs for selection marker into the cells.

In this example, step [1-1] and step [1-2] were carried out simultaneously.

### [Step 2-1]

Among the two types of positive selection marker genes, a gRNA targeting the blasticidin resistance gene was used.

The sequence of the gRNA was completely identical to the target sequence. An expression vector encoding the gRNA was used for introduction of the gRNA. A lipofection method was used for introduction of the gRNA and Cas9 into the cells.

### [Step 2-2]

The upstream homology arm sequence was designed targeting an upstream sequence in the target region (chr6: 29,704,906-29,707,018 of hg38 genome sequence) present in one of two chromosomes (the chromosome to be subjected to homologous recombination).

The downstream homology arm sequence was designed targeting a downstream sequence in the target region (chr6: 29,956,471-29,958,642 of hg38 genome sequence) present in one of two chromosomes (the chromosome to be subjected to homologous recombination).

As the SCD coding sequence, the nucleotide sequence described in SEQ ID NO: 5 was used.

The donor DNA for SCD contained the promoter sequence of the alpha chain gene of HLA-A described in SEQ ID NO: 10.

The donor DNA for SCD was a plasmid.

A lipofection method was used for introduction of the donor DNA for SCD into the cells.

In this example, step [2-1] and step [2-2] were carried out simultaneously.

### [Step 3-1]

Among the two types of positive selection marker genes, a gRNA targeting the puromycin resistance gene was used.

The sequence of the gRNA was completely identical to the target sequence.

An expression vector encoding the gRNA was used for introduction of the gRNA.

A lipofection method was used for introduction of the gRNA and Cas9 into the cells.

### [Step 3-2]

The DNA for selection marker excision was a plasmid.

A lipofection method was used for introduction of the DNA for selection marker excision into the cells.

In this example, step [3-1] and step [3-2] were carried out simultaneously.

The modified cells obtained in step [3-2] were used for knock-in of SCT (Example 2-2).

### [Example 2-2: Knock-in of SCT]

The SCT coding sequence was knocked into the "region containing alpha chain genes of HLA-C and HLA-B" of the cells produced in Example 2-1 according to the method described in the aforementioned "2. Method for knocking in SCT." The "region containing alpha chain genes of HLA-C and HLA-B" was a chromosomal region corresponding to chr6: 31,227,286-31,378,780 of the hg38 genome sequence (Target Region 2). In addition, a CRISPR/Cas system was used as a site-specific nuclease.

### Step [1-1]

A CRISPR/Cas9 system using a guide RNA (gRNA) targeting an upstream region of the alpha chain gene of HLA-C (chr6: 31,228,800-31,228,822 of hg38 genome sequence), a gRNA targeting a downstream region of the alpha chain gene of HLA-B (chr6: 31,377,258-31,377,277 of hg38 genome sequence), and a Cas9 protein was used. The sequences of the gRNAs were completely identical to the target sequences. An expression vector was used for introduction of the gRNA.

A lipofection method was used for introduction of the gRNA and Cas9 into the cells.

### Step [1-2]

The upstream homology arm sequence was designed targeting an upstream sequence in the target region (chr6: 31,227,286-31,228,805 of hg38 genome sequence) present in one of two chromosomes (the chromosome to be subjected to homologous recombination).

The downstream homology arm sequence was designed targeting a downstream sequence in the target region (chr6: 31,377,261-31,378,780 of hg38 genome sequence) present in one of two chromosomes (the chromosome to be subjected to homologous recombination).

The following combinations of selection marker genes possessed by two types of donor DNAs for selection marker were used.

| | Positive selection marker | Negative selection marker |
|---|---|---|
| One donor DNA for selection marker | Puromycin resistance gene | Green fluorescent protein |
| Other donor DNA for selection marker | Blasticidin resistance gene | Red fluorescent protein |

The donor DNAs for selection marker were plasmids.

A lipofection method was used for introduction of the donor DNAs for selection marker into the cells.

In this example, step [1-1] and step [1-2] were carried out simultaneously.

### [Step 2-1]

Among the two types of positive selection marker genes, a gRNA targeting the blasticidin resistance gene was used.

The sequence of the gRNA was completely identical to the target sequence.

An expression vector encoding the gRNA was used for introduction of the gRNA.

A lipofection method was used for introduction of the gRNA and Cas9 into the cells.

### [Step 2-2]

The upstream homology arm sequence was designed targeting an upstream sequence in the target region (chr6: 31,227,286-31,228,805 of hg38 genome sequence) present in one of two chromosomes (the chromosome to be subjected to homologous recombination).

The downstream homology arm sequence was designed targeting a downstream sequence in the target region (chr6: 31,377,261-31,378,780 of hg38 genome sequence) present in one of two chromosomes (the chromosome to be subjected to homologous recombination).

As the SCT coding sequence, the nucleotide sequence described in SEQ ID NO: 7 was used.

The donor DNA for SCD contained the promoter sequence of the alpha chain gene of HLA-B described in SEQ ID NO: 11.

The donor DNA for SCD was a plasmid.

A lipofection method was used for introduction of the donor DNA for SCD into the cells.

In this example, step [2-1] and step [2-2] were carried out simultaneously.

### [Step 3-1]

Among the two types of positive selection marker genes, a gRNA targeting the puromycin resistance gene was used.

The sequence of the gRNA was completely identical to the target sequence. An expression vector encoding the gRNA was used for introduction of the gRNA. A lipofection method was used for introduction of the gRNA and Cas9 into the cells.

### [Step 3-2]

The DNA for selection marker excision was a plasmid.

A lipofection method was used for introduction of the DNA for selection marker excision into the cells.

In this example, step [3-1] and step [3-2] were carried out simultaneously.

In the cells after gene modification obtained in this example, it was confirmed by a PCR method based on the presence or absence of a band showing amplification of the target region that each gene of HLA-F, HLA-G, HLA-A, HLA-C and HLA-B was deleted, and that the polynucleotide sequences encoding SCD and SCT were introduced.

In the cells after gene modification obtained in [Example 2-1: Knock-in of SCD], deletion of each gene of HLA-F, HLA-G and HLA-A and introduction of the polynucleotide sequence encoding SCD were confirmed by the PCR method.

In the cells after gene modification obtained in [Example 2-2: Knock-in of SCT], deletion of each gene of HLA-B and HLA-C and introduction of the polynucleotide sequence encoding SCT were confirmed by the PCR method.

### [Example 3: Expression analysis of gene-modified cell produced in Example 2]

Expression of HLA-A, HLA-B, HLA-C, SCD and SCT in the gene-modified cell produced in Example 2 (hereinafter, "gene-modified cell") was analyzed.

### [Example 3-1: Expression analysis of HLA-A, HLA-B and HLA-C]

Cell surface expression levels of HLA-A, HLA-B and HLA-C in the gene-modified cells were analyzed using a flow cytometry method.

A suspension of the gene-modified cells or control cells (human iPS cell line 201B7 before gene modification) was mixed with a primary antibody recognizing HLA-A, HLA-B and HLA-C (Company name: BioLegend, Catalog number: 311415, Clone number: W6/32), and reacted on ice for 30 minutes.

After the reaction, washing was performed with a washing solution (2% BSA-mixed PBS solution), and samples containing only cells to which the primary antibody bound were collected.

Subsequently, each sample was reacted with a fluorescently labeled antibody (secondary antibody) recognizing the primary antibody, and subjected to a flow cytometer (Thermo Fisher Science, Attune) to analyze the cell surface expression levels of HLA-A, HLA-B and HLA-C.

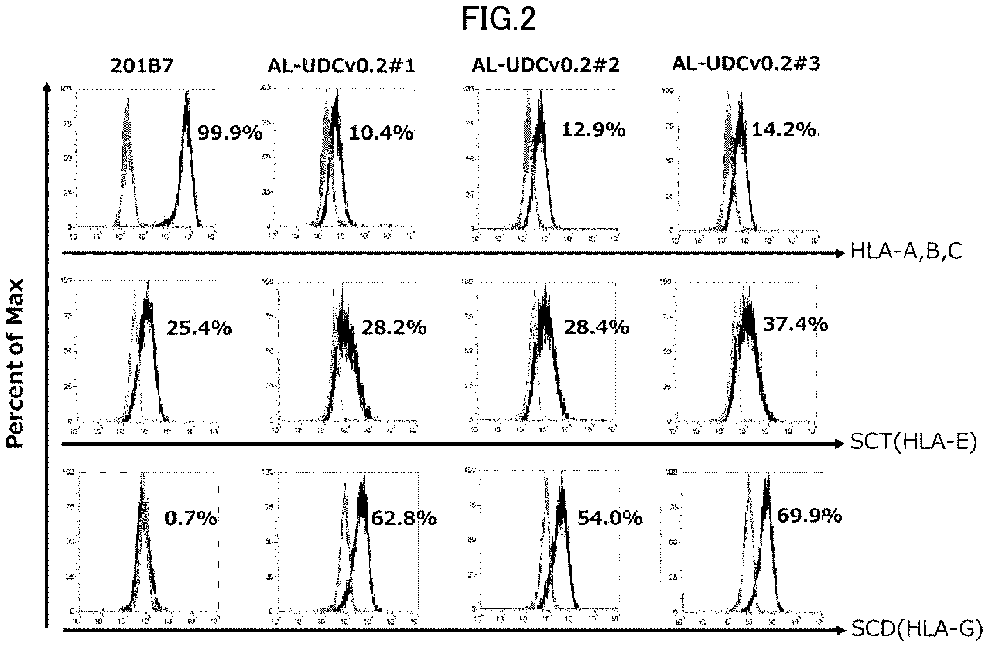

The results are shown in the column of "HLA-A, B, C" in the upper part of Fig. 2. Fig. 2 includes results (panels) of the control cell sample (201B7) and three samples of the gene-modified cells (AL-UDC v0.2 #1 to #3).

Each panel includes two experimental results (IC (gray) and IFN-g (+) (black)).

IC (Isotype control) shows results obtained using a fluorescently labeled primary antibody instead of the primary antibody. IC was used to measure the rate of nonspecific binding of the primary antibody.

"IFN-g (+)" shows results when IFN gamma was added. In this sample, the cells were seeded at 13,000 cells/well, cultured in Medium AK03N (Ajinomoto Healthy Supply Co., Inc.) for 5 days, stimulated with 50 ng/mL IFN-gamma, and additionally cultured for 2 days, and then subjected to expression analysis.

IFN gamma is a substance that induces expression of HLA-A, HLA-B and HLA-C.

The horizontal axis of the figure shows fluorescence intensity. The vertical axis of the figure (Percent of Max) shows the ratio to the fluorescence intensity exhibited by the largest number of cells in the sample.

% in the figure shows the ratio of the number of cells expressing each protein (positive cells).

In the control cell sample (201B7), the histogram significantly shifted to the right side (direction in which fluorescence intensity increases) due to the addition of IFN gamma. This indicates that the expression levels of HLA-A, HLA-B and HLA-C increased due to the addition of IFN gamma.

On the other hand, in the gene-modified cell samples (AL-UDC v0.2 #1 to #3), the degree of shift of the histogram to the right side due to the addition of IFN gamma was small. This indicates that the expression levels of HLA-A, HLA-B and HLA-C did not increase even with the addition of IFN gamma. This result indicates that HLA-A, HLA-B and HLA-C are no longer expressed due to "knock-in of SCD into the region containing the alpha chain gene of HLA-A" and "knock-in of SCT into the region containing alpha chain genes of HLA-C and HLA-B" in the gene-modified cells.

### [Example 3-2: Expression analysis of SCD and SCT]

Cell surface expression levels of SCD and SCT in the gene-modified cells were analyzed using a flow cytometry method.

Expression analysis was performed according to the same procedure as in Example 3-1, except that an anti-HLA-G antibody (abcam, catalog number: ab24384, clone number: MEM-G/9) was used as a primary antibody recognizing SCD (single-chain fusion peptide mimicking HLA-G), and an anti-HLA-E antibody (abcam, catalog number: ab272335, clone number: 3D12) was used as a primary antibody recognizing SCT (single-chain fusion peptide mimicking HLA-E).

The results regarding SCD are shown in the "SCD" column in the lower part of Fig. 2. The results regarding SCT are shown in the "SCT" column in the middle part of Fig. 2.

Regarding SCD, in the control cell sample (201B7), shift of the histogram did not occur even with the addition of IFN gamma.

On the other hand, in the gene-modified cell samples (AL-UDC v0.2 #1 to #3), the histogram significantly shifted to the right side (direction in which fluorescence intensity increases) due to the addition of IFN gamma. This indicates that, in the gene-modified cells in which HLA-G expression (due to the gene possessed by the cells before modification) is lost by "knock-in of SCD into the region containing the alpha chain gene of HLA-G," the promoter sequence of the alpha chain gene of HLA-A (SEQ ID NO: 10) knocked in by the donor DNA for SCD was stimulated by the addition of IFN gamma, and the expression level of downstream SCD (single-chain fusion peptide mimicking HLA-G) increased. Therefore, this result indicates that SCD is knocked in and expressed in the gene-modified cells.

Regarding SCT, in both the control cell sample (201B7) and the gene-modified cell samples (AL-UDC v0.2 #1 to #3), the histogram significantly shifted to the right side (direction in which fluorescence intensity increases) due to the addition of IFN gamma. This indicates that the promoter sequence of the alpha chain gene of HLA-B (SEQ ID NO: 11) knocked in by the donor DNA for SCT was stimulated by the addition of IFN gamma, and the expression level of downstream SCT increased. Note that in the gene-modified cells, although the alpha chain gene of HLA-E remains, the leader sequence of the alpha chain gene of HLA-E is not translated due to the knockout of the alpha chain genes of HLA-A, HLA-B and HLA-C; therefore, the alpha chain of HLA-E is not expressed on the cell surface. Therefore, this result indicates that SCT is knocked in and expressed in the gene-modified cells.

### [Example 3-3: Responsiveness of T cells to gene-modified cells]

T cells recognize self/non-self based on HLA Class I molecules (HLA-A, HLA-B and HLA-C) expressed by target cells. T cells that have recognized target cells as non-self are activated, divide and proliferate, and strengthen cell-mediated immunity (attack on target cells). Utilizing this property of T cells, responsiveness of T cells to the gene-modified cells was evaluated using proliferation of T cells when T cells and the gene-modified cells were co-cultured as an index.

The proliferation of T cells was evaluated using fluorescence intensity of a fluorescent dye CytelRed as an index. When T cells having incorporated this fluorescent dye divide, the fluorescence intensity decreases in the T cells after division.

Before the start of co-culture, T cells (human T lymphocytes; LONZA, catalog number 2W-300) were stained with the fluorescent dye CytelRed (AAT Bioquest, catalog number 22255). The T cell sample at the time of staining (at the time of thawing) was subjected to flow cytometry to measure the ratio of live cells.

The criterion for judgment of live cells was negative for 7-AAD antibody binding to dead cells (7-AAD(-)) and positive for anti-CD8 antibody (CD8(+)) (CD8 is an index of T cells exhibiting cytotoxicity).

As evaluation cells, in addition to the gene-modified cells, human iPS cell line 201B7 (parent strain of the gene-modified cells; hereinafter, "WT cells") and human iPS cell line 201B7 in which B2M gene was deleted (hereinafter, "B2MKO cells") were used.

The evaluation cells and T cells were co-cultured for 10 days. The cell sample after co-culture was subjected to flow cytometry to measure fluorescence intensity, and compared with the result at the time of thawing.

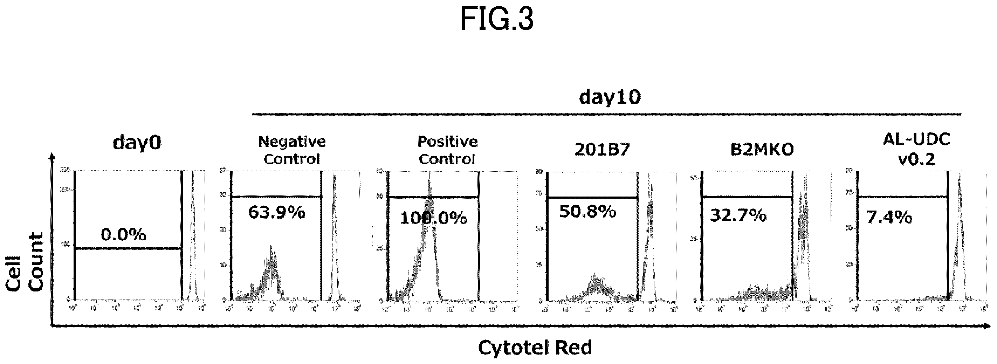

The results are shown in Fig. 3. Fig. 3 includes results (panels) of the T cell sample at the start of culture (Day0), the co-culture sample with gene-modified cells (AL-UDC v0.2), the co-culture sample with WT cells (201B7), the co-culture sample with B2MKO cells (B2MKO), positive control (PC), and negative control (NC).

The horizontal axis of the figure shows the fluorescence intensity of CytelRed. The vertical axis of the figure (Count) shows the number of cells.

% in the figure shows the ratio of divided T cells.

In PC, T cells stimulated with 5 ug/mL anti-CD3 antibody (OKT3) (Invitrogen, catalog number 16-0037-85) cultured alone for 10 days were evaluated. The anti-CD3 antibody binds to the surface antigen CD3 of T cells to activate T cells and promote proliferation. Since a decrease in fluorescence intensity of CytelRed (i.e., proliferation of T cells) occurred in PC, it was confirmed that this test system was established.

In NC, T cells not stimulated with the anti-CD3 antibody cultured alone for 10 days were evaluated. In NC, a decrease in fluorescence intensity of CytelRed did not occur. This indicates that proliferation of T cells did not occur.

In the WT cell co-culture group, a cell population with low fluorescence intensity was observed compared with Day 0 of culture. This indicates that T cells were activated by recognizing WT cells expressing HLA Class I molecules (HLA-A, HLA-B and HLA-C) as non-self, and proliferated.

In the B2MKO cell co-culture group, the fluorescence intensity did not change compared with T cells on Day 0 of culture. This indicates that T cells did not recognize B2MKO cells (in which expression of HLA Class I molecules is suppressed due to deficiency of the gene encoding B2M which is a constituent component of HLA Class I molecules) as non-self, and were not activated (did not proliferate).

Also in the gene-modified cell group, the fluorescence intensity was comparable to that of T cells on Day 0 of culture. This indicates that T cells did not recognize the gene-modified cells (in which expression of HLA Class I molecules is suppressed due to "knock-in of SCD into the region containing the alpha chain gene of HLA-A" and "knock-in of SCT into the region containing alpha chain genes of HLA-C and HLA-B") as non-self, and were not activated (did not proliferate).

The results of this example support that the HLA gene-modified cells of the present invention have a property of avoiding attacks from T cells.

### [Example 3-4: T cell cytotoxicity against gene-modified cells]

In this example, T cell cytotoxicity against the gene-modified cells was evaluated using an impedance assay.

The impedance assay utilizes the property that live cells adhere onto an electrode, spread, and proliferate. When live cells on the electrode proliferate and the number of cells increases, it becomes difficult for current to flow across the entire electrode, and the electrical resistance value (impedance) of the electrode increases. On the other hand, when live cells are killed by T cell injury and the number of live cells present on the electrode decreases, the impedance decreases. This assay indirectly evaluates T cell cytotoxicity against evaluation cells using the change in impedance as an index.

Primary T cells (LONZA, catalog number 2W-300) were seeded at 6 x 10⁴ cells/well in wells coated with 5 ug/mL anti-CD3 antibody (Invitrogen, catalog number 16-0037-85) which is a T cell activator, and cultured with Optimizer medium (Thermo Fisher) containing 200 U/mL IL-2 (PeproTech, catalog number 200-02) for 7 days to produce activated T cells (effector cells).

As target cells, gene-modified cells, WT cells and B2MKO cells were used in the same manner as in Example 3-1.

On the day before the execution of co-culture, the target cells (12,500 cells/well) were seeded on wells equipped with electrodes, and on the next day, the activated T cells were added and co-cultured for 3 days. The impedance value was measured over time with a real-time cell analyzer (xCELLigence). From the measured value, Cell Index (index of cell number) was determined.

The assay was carried out by setting the ratio (E/T ratio) of the number of effector (E) cells to the number of target (T) cells to 10 and 5.

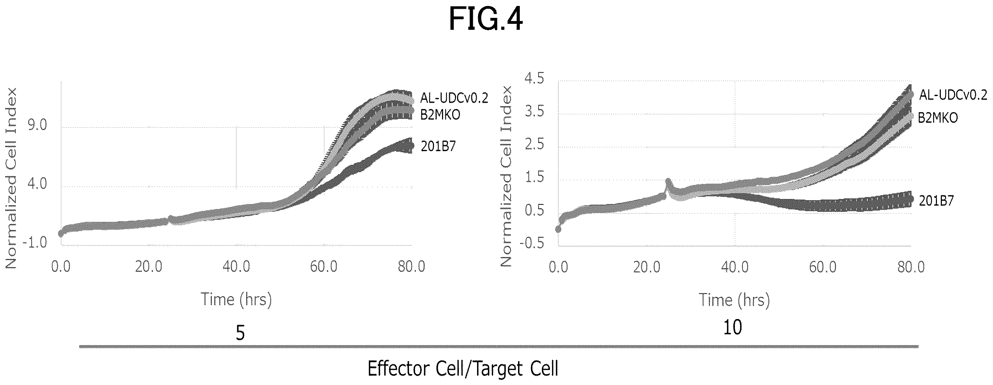

The results are shown in Fig. 4.

At any E/T ratio, compared with the gene-modified cells (AL-UDC v0.2) and B2MKO cells, the degree of increase in Cell Index over time of the WT cells (201B7) was low. This indicates that T cells recognized WT cells expressing HLA Class I molecules (HLA-A, HLA-B and HLA-C) as non-self and attacked (injured) them, whereby a part of WT cells died and the proliferation thereof was suppressed.

On the other hand, the gene-modified cells and B2MKO cells in which expression of HLA Class I molecules (HLA-A, HLA-B and HLA-C) was suppressed proliferated with attenuated injury from T cells.

The results of this example support that the HLA gene-modified cells of the present invention have a property of avoiding attacks from T cells.

### [Example 3-5: NK cell cytotoxicity against gene-modified cells]

In this example, NK cell cytotoxicity against the gene-modified cells was evaluated using an impedance assay.

Regarding the relationship between NK cells and HLA Class I molecules of target cells, when HLA-G expressed on target cells binds to an inhibitory receptor (for example, KIR2DL4 or LILRB1) expressed on NK cells, the cytotoxicity of NK cells is suppressed. Further, when HLA-E expressed on target cells binds to an inhibitory receptor (for example, CD94/NKG2A or CD94/NKG2B) expressed on NK cells, the cytotoxicity of NK cells is suppressed.

Primary NK cells (LONZA, catalog number 2W-501) subjected to flask culture for 6 days using Expansion Kit (R&D Systems, catalog number CDK015) were used as effector cells.

As target cells, gene-modified cells, WT cells and B2MKO cells were used in the same manner as in Example 3-1.

On the day before the execution of co-culture, the target cells (12,500 cells/well) were seeded on wells equipped with electrodes, and on the next day, the NK cells were added and co-cultured for 3 days. The impedance value was measured over time with a real-time cell analyzer (xCELLigence). From the measured value, Cytolysis (%) (index of target cell lysis by NK cell injury) was determined.

The assay was carried out by setting the ratio (E/T ratio) of the number of effector (E) cells to the number of target (T) cells to 1.25, 2.5 and 5.

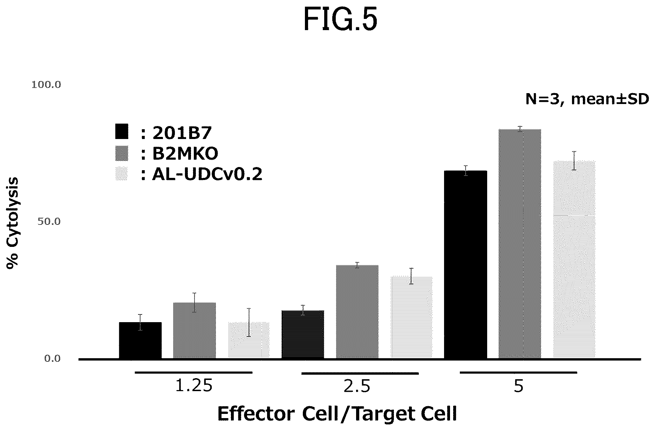

The results are shown in Fig. 5.

At any E/T ratio, Cytolysis (%) of B2MKO cells was higher than that of WT cells (201B7). This indicates that injury by NK cells was greater in B2MKO cells in which expression of HLA-E and HLA-G was suppressed due to deficiency of the B2M coding gene than in WT cells.

On the other hand, under conditions where the E/T ratio was 2.5 and 5, Cytolysis (%) of the gene-modified cells (AL-UDC v0.2) was lower than that of B2MKO cells. This indicates that cytotoxicity by NK cells was suppressed by expression of SCD (single-chain fusion peptide mimicking HLA-G) and SCT (single-chain fusion peptide mimicking HLA-E).

The results of this example support that the HLA gene-modified cells of the present invention have a property of avoiding attacks from NK cells.

### [Example 4: Production of HLA gene-modified cell in which region containing alpha chain genes of HLA-F, HLA-G and HLA-A was replaced (knocked in) by SCD coding sequence, region containing alpha chain genes of HLA-C and HLA-B was replaced (knocked in) by SCT coding sequence, and region containing CIITA gene was deleted (knocked out)]

"4. Method for knocking out CIITA gene" was carried out on the gene-modified cells produced in the aforementioned Example 2 to produce CIITA gene-deleted cells.

### [Knockout of CIITA gene]

The "region containing CIITA gene" of the cells was knocked out according to the method described in the aforementioned "4. Method for knocking out CIITA gene." The "region containing CIITA gene" was a chromosomal region corresponding to the chromosomal region of chr16: 10,873,026-10,925,966 of the hg38 genome sequence (Target Region 2). In addition, a CRISPR/Cas system was used as a site-specific nuclease.

### Step [1-1]

A CRISPR/Cas9 system using a guide RNA (gRNA) targeting an upstream region of the CIITA gene (chr16: 10,873,824-10,873,846 of hg38 genome sequence), a gRNA targeting a downstream region of the CIITA gene (chr16: 10,925,149-10,925,171 of hg38 genome sequence), and a Cas9 protein was used.

The sequences of the gRNAs were completely identical to the target sequences.

An expression vector was used for introduction of the gRNA.

A lipofection method was used for introduction of the gRNA and Cas9 into the cells.

### Step [1-2]

The upstream homology arm sequence was designed targeting an upstream sequence in the target region (chr16: 10,873,026-10,873,829 of hg38 genome sequence) present in one of two chromosomes (the chromosome to be subjected to homologous recombination).

The downstream homology arm sequence was designed targeting a downstream sequence in the target region (chr16: 10,925,155-10,925,966 of hg38 genome sequence) present in one of two chromosomes (the chromosome to be subjected to homologous recombination).

The following combinations of selection marker genes possessed by two types of donor DNAs for selection marker were used.

| | Positive selection marker | Negative selection marker |
|---|---|---|
| One donor DNA for selection marker | Puromycin resistance gene | Green fluorescent protein |
| Other donor DNA for selection marker | Blasticidin resistance gene | Red fluorescent protein |

The donor DNAs for selection marker were plasmids.

A lipofection method was used for introduction of the donor DNAs for selection marker into the cells.

In this example, step [1-1] and step [1-2] were carried out simultaneously.

### [Step 2-1]

Two types of gRNAs targeting each of the two types of positive selection marker genes used in step [1-2] were used.

The sequences of the gRNAs were completely identical to the target sequences.

An expression vector encoding the gRNA was used for introduction of the gRNA.

A lipofection method was used for introduction of the gRNA and Cas9 into the cells.

### [Step 2-2]

For the "upstream homology arm sequence" and "downstream homology arm sequence" of the DNA for selection marker excision, the same sequences as the "upstream homology arm sequence" and "downstream homology arm sequence" of the donor DNA for selection marker used in step [1-2] were used, respectively.

A plasmid carrying the DNA was used for introduction of the DNA for selection marker excision.

A lipofection method was used as a method for introducing the DNA for selection marker excision into the cells.

In this example, step [2-1] and step [2-2] were carried out simultaneously.

In the cells after gene modification obtained in this example, it was confirmed by a PCR method that the CIITA gene was deleted by the fact that no band showing amplification of the target region appeared.

In the cells after gene modification obtained in this example, in addition to the deletion of HLA Class I genes and the introduction of SCD and SCT in the gene-modified cells produced in Example 2, expression of HLA Class II molecules is suppressed due to deletion of the CIITA gene.

Suppression of expression of HLA Class II molecules is confirmed by culturing cells obtained by differentiating the cells after gene modification obtained in this example (for example, cells differentiated into mesenchymal stem cells, or cells differentiated into antigen-presenting cells such as B cells, monocytes, macrophages, dendritic cells, and Langerhans cells) for a period sufficient for maturation of the differentiated cells, and evaluating expression of mRNA by a qPCR method or evaluating expression of HLA Class II proteins by a flow cytometry method. For evaluation of expression of HLA Class II proteins by the flow cytometry method, an antibody recognizing HLA Class II (for example, an antibody against anti-HLA-DR/DQ/DP) is used. Evaluation of expression of HLA Class II molecules can also be carried out for each condition with or without induction by IFN gamma.

### Industrial Applicability

The present invention is applicable in the field of cell-based regenerative medicine.

## Claims

1. An HLA gene-modified cell, wherein polynucleotides of the following (1) and (2) are knocked into a region containing alpha chain genes of HLA-A, HLA-B, HLA-C, HLA-F and HLA-G:
(1) a polynucleotide encoding a single-chain dimer (SCD) fusion peptide comprising beta-2 microglobulin and an alpha chain of HLA-G;
(2) a polynucleotide encoding a single-chain trimer (SCT) fusion peptide comprising a signal peptide of HLA-A, HLA-B, HLA-C or HLA-G, beta-2 microglobulin, and an alpha chain of HLA-E.

2. The cell according to claim 1, wherein expression of alpha chain genes of HLA-A, HLA-B, HLA-C, HLA-F and HLA-G is reduced compared with a wild-type cell.

3. The cell according to claim 1, wherein alpha chain genes of HLA-A, HLA-B, HLA-C, HLA-F and HLA-G of the cell before gene modification are deleted.

4. The cell according to claim 1, wherein
(1) the polynucleotide encoding SCD is knocked into a region containing alpha chain genes of HLA-F, HLA-G and HLA-A, and
(2) the polynucleotide encoding SCT is knocked into a region containing alpha chain genes of HLA-C and HLA-B.

5. The cell according to claim 1, wherein
(2) the polynucleotide encoding SCT is knocked into a region containing alpha chain genes of HLA-F, HLA-G and HLA-A, and
(1) the polynucleotide encoding SCD is knocked into a region containing alpha chain genes of HLA-C and HLA-B.

6. An HLA gene-modified cell, wherein a polynucleotide of the following (1) is knocked into a region containing alpha chain genes of HLA-F, HLA-G and HLA-A:
(1) a polynucleotide encoding a single-chain dimer (SCD) fusion peptide comprising beta-2 microglobulin and an alpha chain of HLA-G.

7. The cell according to claim 6, wherein expression of alpha chain genes of HLA-F, HLA-G and HLA-A is reduced compared with a wild-type cell.

8. The cell according to claim 6, wherein alpha chain genes of HLA-F, HLA-G and HLA-A of the cell before gene modification are deleted.

9. An HLA gene-modified cell, wherein a polynucleotide of the following (2) is knocked into a region containing alpha chain genes of HLA-C and HLA-B:
(2) a polynucleotide encoding a single-chain trimer (SCT) fusion peptide comprising a signal peptide of HLA-A, HLA-B, HLA-C or HLA-G, beta-2 microglobulin, and an alpha chain of HLA-E.

10. The cell according to claim 9, wherein expression of alpha chain genes of HLA-C and HLA-B is reduced compared with a wild-type cell.

11. The cell according to claim 9, wherein alpha chain genes of HLA-C and HLA-B of the cell before gene modification are deleted.

12. The cell according to claim 1 or 6, wherein the region containing alpha chain genes of HLA-F, HLA-G and HLA-A is a chromosomal region corresponding to a chromosomal region of chr6: 29,704,906-29,958,642 of hg38 genome sequence.

13. The cell according to claim 1 or 9, wherein the region containing alpha chain genes of HLA-C and HLA-B is a chromosomal region corresponding to a chromosomal region of chr6: 31,224,837-31,378,196 of hg38 genome sequence.

14. The cell according to claim 1 or 9, wherein the region containing alpha chain genes of HLA-C and HLA-B is a chromosomal region corresponding to a chromosomal region of chr6: 31,227,286-31,378,780 of hg38 genome sequence.

15. The cell according to claim 1, 6 or 9, wherein (1) the polynucleotide encoding SCD and/or (2) the polynucleotide encoding SCT is knocked into only one allele of a subject cell.

16. The cell according to claim 1, 6 or 9, wherein expression of an HLA Class II molecule gene is further reduced compared with a wild-type cell by modification.

17. The cell according to claim 1, 6 or 9, wherein an HLA Class II molecule gene is further deleted by modification.

18. The cell according to claim 16, wherein the HLA Class II molecule gene is a CIITA gene.

19. The cell according to claim 17, wherein the HLA Class II molecule gene is a CIITA gene.

20. The cell according to claim 1, 6 or 9, which is a human pluripotent stem cell.

21. The cell according to claim 1, 6 or 9, which is a human iPS cell or ES cell.

22. The cell according to claim 1, 6 or 9, which is a human iPS cell line 253G4 or a human iPS cell line 201B7.

23. The cell according to claim 1, 6 or 9, which is a cell obtained by differentiating a human pluripotent stem cell.

24. The cell according to claim 23, wherein the human pluripotent stem cell is a human iPS cell or ES cell.

25. The cell according to claim 23, wherein the human pluripotent stem cell is a human iPS cell line 253G4, a human iPS cell or ES cell.

26. The cell according to claim 23, wherein the cell obtained by differentiating a human pluripotent stem cell is an immune cell, a tissue stem cell or a somatic cell.

27. The cell according to claim 26, wherein the immune cell is a T cell or an NK cell.

28. The cell according to claim 1 or 6, wherein the SCD comprises an amino acid sequence set forth in SEQ ID NO: 1 or 4, or an amino acid sequence having 80% or more sequence identity with the amino acid sequence set forth in SEQ ID NO: 1 or 4.

29. The cell according to claim 1 or 9, wherein the SCT comprises an amino acid sequence set forth in SEQ ID NO: 6, or an amino acid sequence having 80% or more sequence identity with the amino acid sequence set forth in SEQ ID NO: 6.

30. The cell according to claim 1 or 6, wherein the polynucleotide of (1) comprises a nucleotide sequence set forth in SEQ ID NO: 2 or 5, or a nucleotide sequence having 80% or more sequence identity with the nucleotide sequence set forth in SEQ ID NO: 2 or 5.

31. The cell according to claim 1 or 9, wherein the polynucleotide of (2) comprises a nucleotide sequence set forth in SEQ ID NO: 7, or a nucleotide sequence having 80% or more sequence identity with the nucleotide sequence set forth in SEQ ID NO: 7.

32. The cell according to claim 1 or 6, wherein the SCD further comprises a linker sequence between the beta-2 microglobulin and the alpha chain of HLA-G.

33. The cell according to claim 1 or 9, wherein the SCT further comprises a linker sequence between the signal peptide and the beta-2 microglobulin.

34. The cell according to claim 1 or 9, wherein the SCT further comprises a linker sequence between the beta-2 microglobulin and the alpha chain of HLA-E.

35. The cell according to claim 32, wherein the linker sequence comprises a peptide consisting of glycine and serine.

36. The cell according to claim 33, wherein the linker sequence comprises a peptide consisting of glycine and serine.

37. The cell according to claim 34, wherein the linker sequence comprises a peptide consisting of glycine and serine.

38. The cell according to claim 32, wherein the linker sequence comprises an amino acid sequence set forth in SEQ ID NO: 3.

39. The cell according to claim 33, wherein the linker sequence comprises an amino acid sequence set forth in SEQ ID NO: 8.

40. The cell according to claim 34, wherein the linker sequence comprises an amino acid sequence set forth in SEQ ID NO: 9.

41. The cell according to claim 1 or 6, wherein a promoter sequence of an alpha chain gene of HLA-A is knocked in together with the polynucleotide of (1), and the promoter sequence is operably linked to a sequence encoding SCD.

42. The cell according to claim 41, wherein the promoter sequence of the alpha chain gene of HLA-A comprises a nucleotide sequence set forth in SEQ ID NO: 10, or a nucleotide sequence having 80% or more sequence identity with the nucleotide sequence set forth in SEQ ID NO: 10.

43. The cell according to claim 1 or 9, wherein a promoter sequence of an alpha chain gene of HLA-B is knocked in together with the polynucleotide of (2), and the promoter sequence is operably linked to a sequence encoding SCT.

44. The cell according to claim 43, wherein the promoter sequence of the alpha chain gene of HLA-B comprises a nucleotide sequence set forth in SEQ ID NO: 11, or a nucleotide sequence having 80% or more sequence identity with the nucleotide sequence set forth in SEQ ID NO: 11.

45. The cell according to claim 1, 6 or 9, wherein rejection reaction by T cells and/or NK cells is alleviated compared with a wild-type cell.

46. The cell according to claim 45, wherein the T cells are CD8⁺ T cells and/or CD4⁺ T cells.

47. A cell derived from the HLA gene-modified cell according to claim 1, 6 or 9.
